# EUROPEAN PATENT APPLICATION

(11) **EP 1 990 414 A1**
(43) Date of publication of application: **12.11.2008**
(21) Application number: 07009444.6
(22) Date of filing: 10.05.2007
(51) Int. Cl.: C12N 15/11

(54) **Means for treating inflammatory diseases, autoimmune diseases and cancer**

(71) Applicant: Fraunhofer-Gesellschaft zur Förderung der angewandten Forschung e.V., 80686 München (DE)
(72) Inventor: Horn, Friedemann, 04316 Leipzig (DE)
(74) Representative: Vossius & Partner

(57) **Abstract**

This invention relates to a compound, wherein said compound is (a) a polynucleotide comprising or consisting of a base sequence of contiguous bases from the sequence of any one of SEQ ID NOs: 1 to 5, said base sequence being at least 15 bases in length; (b) a polynucleotide having at least 80% sequence identity to the polynucleotide of (a) over the entire length of said base sequence; (c) an analog or derivative of (a) or (b); or (d) a polynucleotide or analog or derivative thereof which is complementary to the full length of any one of (a) to (c); wherein said polynucleotide having at least 80% sequence identity, said analog, or said derivative is capable of interacting with a nucleic acid comprising a double-stranded part, said part comprising the sequence of any one of SEQ ID NOs: 1 to 5. The sequences of SEQ ID NOs: 1 to 5 define the Stat3-binding region of the gene encoding the micro RNA miR-21 and homologues thereof.

## Description

This invention relates to a compound, wherein said compound is (a) a polynucleotide comprising or consisting of a base sequence of contiguous bases from the sequence of any one of SEQ ID NOs: 1 to 5, said base sequence being at least 15 bases in length; (b) a polynucleotide having at least 80% sequence identity to the polynucleotide of (a) over the entire length of said base sequence; (c) an analog or derivative of (a) or (b); or (d) a polynucleotide or analog or derivative thereof which is complementary to the full length of any one of (a) to (c); wherein said polynucleotide having at least 80% sequence identity, said analog, or said derivative is capable of interacting with a nucleic acid comprising a double-stranded part, said part comprising the sequence of any one of SEQ ID NOs: 1 to 5.

In this specification, a number of documents including patent applications and manufacturers' manuals is cited. The disclosure of these documents, while not considered relevant for the patentability of this invention, is herewith incorporated by reference in its entirety.

*Signal transducer and activator of transcription* 3 *(Stat3),* an intracellular signalling protein activated by various cytokines, hormones and growth factors, directly regulates the transcription of specific target genes and thereby controls the cell physiology with respect to cell cycle, apoptosis (programmed cell death), differentiation and other processes. Stat3 is critically involved in the pathogenesis of various diseases. The factor is known to participate in cellular transformation and oncogenesis (1, 2). Its activation is essential for the transforming potential of a number of oncogenes (3). For an increasing number of cancer types, including multiple myeloma, Hodgkin lymphoma, glioblastoma, and colon, prostate, pancreas, mammary carcinoma and others, Stat3 is considered as being involved in the control of cell growth and survival (4-11). Remarkably, constitutive Stat3 activation has proven in various malignancies to be a marker for short survival and/or high tumour invasiveness and high incidence of metastases (12-14). Hence, Stat3 hyperactivation represents a molecular status of tumours and leukemias defining subgroups of such diseases with particularly poor prognosis (15-18). In addition, Stat3 is implicated in the control of differentiation processes in many immune cells and thereby plays a pivotal role for the immune system. In that context, Stat3 is involved in the pathogenesis of immune-related diseases like chronic inflammatory and autoimmune diseases as well as the immune tolerance against tumour cells (2, 19-22). Accordingly, Stat3 has been recognised as a highly promising therapeutic target for such diseases (9, 23, 24).

*MicroRNAs (miRNAs)* are an abundant class of short non-protein-coding RNAs that mediate posttranscriptional down-regulation of target genes (25). They have been shown to play a fundamental role in diverse physiological and pathological processes including proliferation, apoptosis (26, 27), and differentiation (28, 29). Recent evidence indicates that miRNAs can function as tumour suppressor genes and oncogenes (30, 31). Among them, *miRNA-21 (miR-21)* has been described to be overexpressed in glioblastoma and breast cancer and to exhibit antiapoptotic and proliferative activity (32-36). The RNA sequence of human miR-21 is 5'-UAGCUUAUCAGACUGAUGUUGA-3' (SEQ ID NO: 6).

The mechanism of miRNA action involves at the one hand actions towards specific target mRNAs, either inducing their degradation or blocking their translation. On the other hand, miRNAs have also been demonstrated to affect the transcriptional process, e.g. by influencing the epigenetic status of chromatin regions. This epigenetic status comprises the modification of DNA and DNA-bound proteins like histones as well as the overall chromatin structure. Epigenetic features critically control the transcriptional activity of a particular gene locus. Thus, by changing such features, miRNAs and other short RNA molecules like small interfering RNAs (siRNAs) are able to directly govern gene transcription.

Transcriptional gene silencing caused by small interfering RNAs (siRNA) was first observed in plants and insects and was indicated to involve DNA methylation of the targeted gene. However, recent reports have documented that siRNAs targeted to genes can induce transcriptional silencing in mammalian cells as well (37, 38). In fact, the successful use of short RNAs to selectively silence gene expression by targeting a promoter element and inducing DNA and/or histone methylation has been demonstrated (39-41).

Targeting Stat3 has been proposed as a therapeutic strategy for the treatment of various diseases. However, Stat3 is an ubiquitously expressed protein that controls the transcription of a large number of genes and thereby regulates physiological processes in numerous cell and organ types. Hence, silencing Stat3 expression or blocking its activity by oligonucleotide-based or small-compound drugs bears a considerable risk of causing multiple adverse side effects.

In view of these limitations of the prior art, the technical problem underlying the present invention was therefore the provision of further or improved means and methods of treating diseases including inflammatory diseases, autoimmune disease and cancer. Expressed in terms of the underlying molecular mechanisms, the technical problem underlying the present invention can be seen in the provision of further or improved means and methods for treating Stat3-dependent diseases.

Accordingly, this invention relates to a compound, wherein said compound is (a) a polynucleotide comprising or consisting of a base sequence of contiguous bases from the sequence of any one of SEQ ID NOs: 1 to 5, said base sequence being at least 15 bases in length; (b) a polynucleotide having at least 80% sequence identity to the polynucleotide of (a) over the entire length of said base sequence; (c) an analog or derivative of (a) or (b); or (d) a polynucleotide or analog or derivative thereof which is complementary to the full length of any one of (a) to (c); wherein said polynucleotide having at least 80% sequence identity, said analog, or said derivative is capable of interacting with a nucleic acid comprising a double-stranded part, said part comprising the sequence of any one of SEQ ID NOs: 1 to 5.

The term "polynucleotide" as used herein refers to a condensation polymer of nucleotides. The term "polynucleotide" according to the invention includes oligonucleotides, wherein oligonucleotides are condensation oligomers of about 30 or less nucleotides. The polynucleotides according to the invention may have 1000, 2000, 5000, 10000 or more bases. In preferred embodiments, in particular in those directed to ribozymes further detailed below, the preferred number of bases in the polynucleotides according to the invention is not more than 500, 400, 300 or 200 bases. In other preferred embodiments, such as antisense molecules or siRNAs (see below), the preferred lengths of the polynucleotide according to the invention is 100 bases or below 100 bases.

It is understood that the term "base sequence" refers to a molecule which, in addition to the nucleobases forming the sequence of nucleobases, i.e. without any linking backbone, also comprises a backbone linking said nucleobases together such as the sugar-phospate backbone in case of standard poly- or oligonucleotides, wherein the term "standard" is meant as a distinction from analogs and derivatives. In other words, said base sequence, while being comprised by the polynucleotide of the invention, may in turn be an oligonucleotide, analog or derivative thereof or, in case said base sequence exceeds a length of about 30 bases, a polynucleotide, analog or derivative thereof.

The term "sequence identity" refers to the number of identical bases in aligned nucleotide sequences expressed as a relative number in percent. These percentage values can be obtained by using bioinformatic tools well-known in the art. To explain further, two nucleotide or protein sequences can be aligned electronically using suitable computer programs known in the art. Such programs comprise BLAST (42), variants thereof such as WU-BLAST (43), FASTA (44) or implementations of the Smith-Waterman algorithm (SSEARCH, (45)). These programs, in addition to providing a pairwise sequence alignment, also report the sequence identity level (usually in percent identity) and the probability for the occurrence of the alignment by chance (P-value). Programs such as CLUSTALW (46) can be used to align more than two sequences.

Preferably, said sequence identity is 85%, 90% or 95%, more preferred 96%, 97% or 99%.

The terms "analog" and "derivative" of polynucleotides are well-known in the art. Preferred embodiments thereof are further detailed below. Furthermore, the analogs and derivatives according to the main embodiment of the invention are limited to those which are capable of interacting with a nucleic acid comprising a double-stranded part, said part comprising the sequence of any one of SEQ ID NOs: 1 to 5. Preferably, this interaction occurs with the double stranded part of said nucleic acid, more preferred with the region in the double stranded part which comprises the sequence of any one of SEQ ID NOs: 1 to 5 as one of the strands.

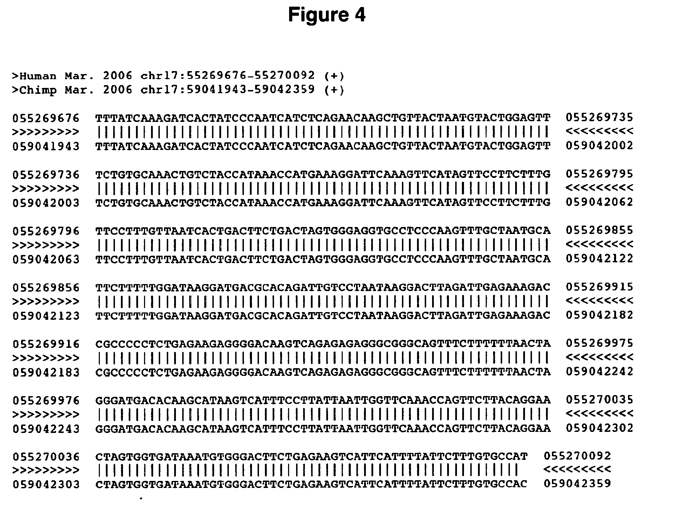

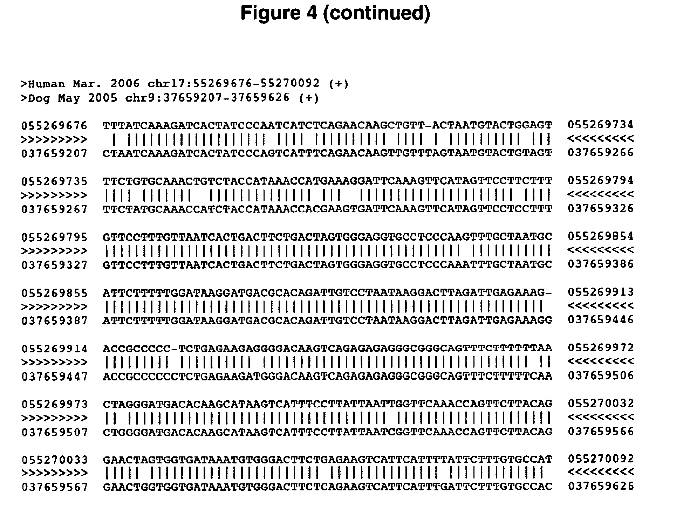

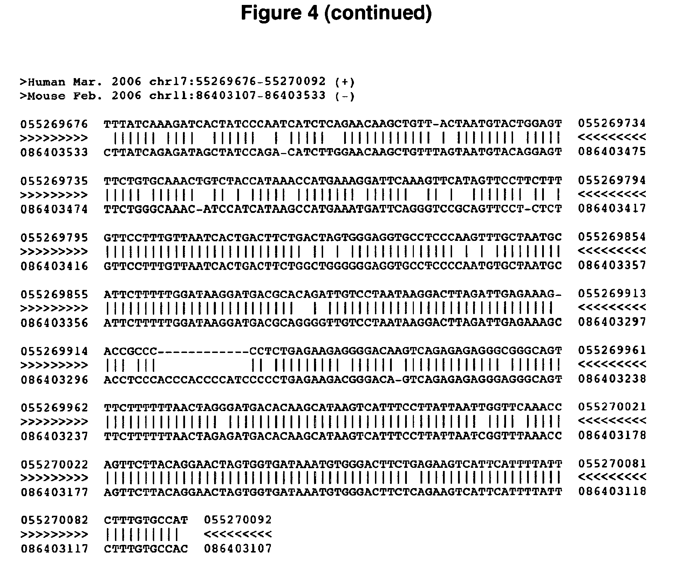

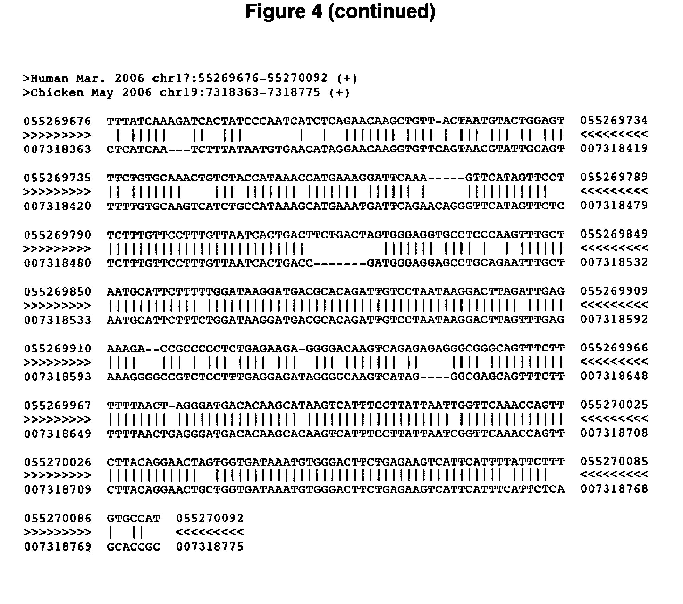

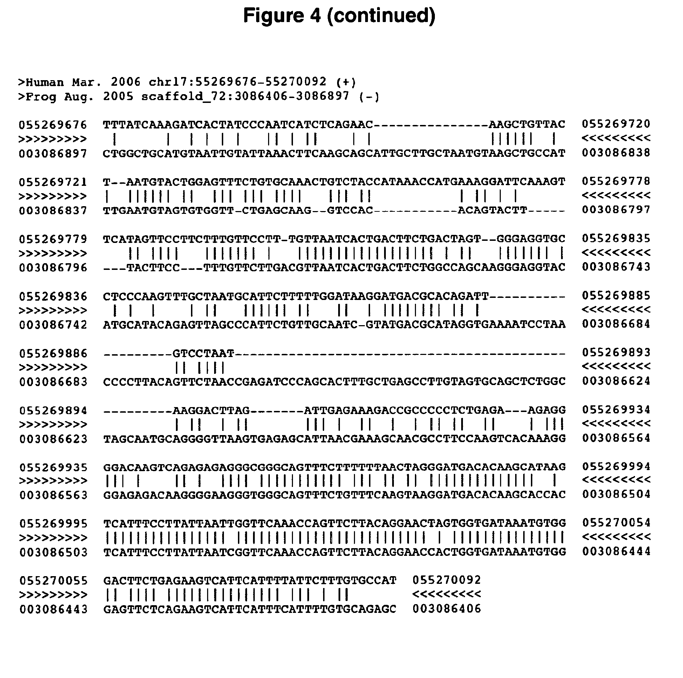

The sequences of SEQ ID NOs: 1 to 5 define the Stat3-binding region of the gene encoding the micro RNA miR-21 and homologues thereof. The compounds according to the main embodiment interact with, preferably bind to this enhancer region. The present inventors found that the gene for miR-21 is under transcriptional control of Stat3. An upstream regulatory element (enhancer) of that gene contains two specific binding sites for Stat3 and is highly conserved in vertebrates (see Fig. 4) suggesting a close functional correlation between the action of Stat3 and the expression of miR-21. This Stat3-controlled expression of miR-21 has been verified experimentally (see Examples enclosed). This observation implies that induction of miR-21 critically contributes to the pathogenetic potential of Stat3. Experimental data strongly support that view (see Examples).

Therefore, targeting the expression and/or function of miR-21 provides a therapeutic strategy for the treatment of diseases whose molecular pathogenesis involves the action of transcription factor Stat3, and will be more selective than blocking Stat3 function *in toto* as it targets only one, yet pathogenetically critical, Stat3 target gene.

In a preferred embodiment of the compound of the invention, said base sequence is not more than 50 bases in length, preferably between 20 and 25 bases, most preferred 22 bases. This embodiment further limits the length of the sequence of contiguous bases to be chosen from the sequence of any one of SEQ ID NOs: 1 to 5. In those embodiments where the nucleotide according to the invention consists of said sequence of contiguous bases, the limitations regarding length of the base sequence according to this preferred embodiment also refer to the polynucleotide according to the invention. In a further preferred embodiment, the length of said base sequence is the length of any one of SEQ ID NOs: 1 to 5. More preferred, said base sequence is the sequence of any one of SEQ ID NOs: 1 to 5.

The present invention also relates to a pharmaceutical composition comprising (a) one or more compounds according to the invention; and/or (b) an inhibitor of the interaction between Stat3 and a nucleic acid as defined in the main embodiment. Said inhibitor is obtainable by the method of screening according to the invention described further below.

The pharmaceutical composition may further comprise pharmaceutically acceptable carriers, excipients and/or diluents. Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. It is particularly preferred that said administration is carried out by injection. The compositions may also be administered directly to the target site, e.g., by biolistic delivery (also referred to as "gene gun") to an external or internal target site. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Pharmaceutically active polynucleotides, nucleic acids or analogs and derivatives thereof may be present in amounts between 1 ng and 10 mg/kg body weight per dose; however, doses below or above this exemplary range are envisioned, especially considering the aforementioned factors. If the regimen is a continuous infusion, it should also be in the range of 1 µg to 10 mg units per kilogram of body weight per minute.

Stat3 is an abbreviation of "signal transducer and activator of transcription 3". The molecule is a well-known intracellular signalling protein activated by various cytokines, hormones and growth factors and directly regulates the transcription of specific target genes and thereby controls the cell physiology with respect to cell cycle, apoptosis (programmed cell death), differentiation and other processes and is further described in the background section herein above. Preferred embodiments of Stat3 are further obtained below.

Furthermore provided by the present invention is the use of one or more compounds of the invention or of the inhibitor defined herein above for the manufacture of a pharmaceutical composition for the treatment of cancer, inflammatory diseases, autoimmune diseases, or a disease with an involvement of Stat3 activation for aetiology and/or progression.

The present inventors presently surprisingly found that Stat3 binds to an enhancer located in a gene encoding miR-21. Thereby a route is opened for the treatment of cancer, inflammatory diseases and autoimmune diseases as well as for other diseases with an involvement of Stat3 activation for aetiology and/progression which involves the formulation of compounds or the above-defined inhibitor into a pharmaceutical composition and the administration of the obtained pharmaceutical composition to a patient in need thereof. Without being bound by specific theory, it is considered that it is the involvement of Stat3 in the recited conditions of cancer, inflammatory diseases and autoimmune diseases which renders these conditions amenable to the treatment with the compounds or the inhibitor according to the invention.

To explain further, for multiple myeloma, an aggressive plasma-cell malignancy, therapeutic targeting of miR-21 is particularly preferred. Stat3 - in these cells activated primarily by the cytokine interleukin-6 (IL-6) - plays a pivotal role in the development of this disease. Growth and survival of primary myeloma cells as well as established myeloma cell lines often is IL-6-dependent, and cells are sent into apoptosis upon either cytokine withdrawal or Stat3 inhibition (11, 47, 48). Experimental evidence now demonstrates that IL-6 induces miR-21 expression in myeloma cells through the abovementioned Stat3-regulated enhancer, and that ectopic overexpression of miR-21 renders the cells at least partially IL-6-independent (see Examples). Since miR-21 is only one among several hundreds of genes that are regulated by Stat3 in myeloma cells (11), inhibiting the expression or function of that miRNA will be a more specific and selective means to interfere with the growth and survival of these cells *in vitro* and *in vivo.* Hence, such a therapeutic strategy is expected to block the growth of or to cause regression of multiple myeloma without the high hazard of adverse side reactions expected upon targeting Stat3.

Analogously, targeting miR-21 is envisaged for the treatment of other malignancies that involve Stat3 action as discussed above. The same considerations apply to non-malignant diseases associated with deregulated Stat3 activity like chronic inflammatory and autoimmune diseases including Morbus Crohn, psoriasis, rheumatoid arthritis and many more.
Immune tolerance against tumour cells is another pathological situation Stat3 is involved in, particularly by its deregulated activity in dendritic cells and monocytes/macrophages in tumour patients. A causal role of miR-21 in these circumstances as well appears likely.

In the following a list of malignant diseases is provided with an involvement of hyper-activated transcription factor Stat3 (references to publications documenting the link between the respective disease and Stat3 are provided in brackets):

Acute myeloid leukemia
(Benekli, M., Z. Xia, K. A. Donohue, L. A. Ford, L. A. Pixley, M. R. Baer, H. Baumann, and M. Wetzler. 2002. Constitutive activity of signal transducer and activator of transcription 3 protein in acute myeloid leukemia blasts is associated with short disease-free survival. Blood 99:252*.)*

Anaplastic large cell lymphoma
(Amin, H. M., T. J. McDonnell, Y. Ma, Q. Lin, Y. Fujio, K. Kunisada, V. Leventaki, P. Das, G. Z. Rassidakis, C. Cutler, L. J. Medeiros, and R. Lai. 2004. Selective inhibition of STAT3 induces apoptosis and G(1) cell cycle arrest in ALK-positive anaplastic large cell lymphoma. Oncogene 23:5426*.)*

Cholangiocarcinoma
(Isomoto, H., S. Kobayashi, N. W. Werneburg, S. F. Bronk, M. E. Guicciardi, D. A. Frank, and G. J. Gores. 2005. Interleukin 6 upregulates myeloid cell leukemia-1 expression through a STAT3 pathway in cholangiocarcinoma cells. Hepatology 42:1329.)

Colon carcinoma (colorectal cancer)
(Kusaba, T., T. Nakayama, K. Yamazumi, Y. Yakata, A. Yoshizaki, K. Inoue, T. Nagayasu, and I. Sekine. 2006. Activation of STAT3 is a marker of poor prognosis in human colorectal cancer. Oncol. Rep. 15:1445*.)*

Glioblastoma
(Rahaman, S. O., P. C. Harbor, O. Chernova, G. H. Barnett, M. A. Vogelbaum, and S. J. Haque. 2002. Inhibition of constitutively active Stat3 suppresses proliferation and induces apoptosis in glioblastoma multiforme cells. Oncogene 21:8404*.)* Head and neck cancer
(Leeman, R. J., V. W. Lui, and J. R. Grandis. 2006. STAT3 as a therapeutic target in head and neck cancer. Expert Opin Biol Ther 6:231*.)*

Hepatocellular carcinoma
(Li, W. C., S. L. Ye, R. X. Sun, Y. K. Liu, Z. Y. Tang, Y. Kim, J. G. Karras, and H. Zhang. 2006. Inhibition of growth and metastasis of human hepatocellular carcinoma by antisense oligonucleotide targeting signal transducer and activator of transcription 3. Clin. Cancer Res. 12:7140*.)*

Hodgkin lymphoma
(Holtick, U., M. Vockerodt, D. Pinkert, N. Schoof, B. Sturzenhofecker, N. Kussebi, K. Lauber, S. Wesselborg, D. Löffler, F. Horn, L. Trümper, and D. Kube. 2005. STAT3 is essential for Hodgkin lymphoma cell proliferation and is a target of tyrphostin AG17 which confers sensitization for apoptosis. Leukemia 19:936*.)*

Lung cancer
(Yeh, H. H., W. W. Lai, H. H. Chen, H. S. Liu, and W. C. Su. 2006. Autocrine IL-6-induced Stat3 activation contributes to the pathogenesis of lung adenocarcinoma and malignant pleural effusion. Oncogene 25:4300*.*
Song, L., J. Turkson, J. G. Karras, R. Jove, and E. B. Haura. 2003. Activation of Stat3 by receptor tyrosine kinases and cytokines regulates survival in human non-small cell carcinoma cells. Oncogene 22:4150*.)*

Mammary carcinoma (breast cancer)
(Ling, X., and R. B. Arlinghaus. 2005. Knockdown of STAT3 expression by RNA interference inhibits the induction of breast tumors in immunocompetent mice. Cancer Res. 65:2532*.)*

Melanoma
(Xie, T. X., F. J. Huang, K. D. Aldape, S. H. Kang, M. Liu, J. E. Gershenwald, K. Xie, R. Sawaya, and S. Huang. 2006. Activation of stat3 in human melanoma promotes brain metastasis. Cancer Res. 66:3188*.)*

Multiple Myeloma
(Catlett-Falcone, R., T. H. Landowski, M. M. Oshiro, J. Turkson, A. Levitzki, R. Savino, G. Ciliberto, L. Moscinski, J. L. Fernandez-Luna, G. Nunez, W. S. Dalton, and R. Jove. 1999. Constitutive activation of Stat3 signaling confers resistance to apoptosis in human U266 myeloma cells. Immunity 10:105*.*
Brocke-Heidrich, K., A. K. Kretzschmar, G. Pfeifer, C. Henze, D. Loffler, D. Koczan, H. J. Thiesen, R. Burger, M. Gramatzki, and F. Horn. 2004. Interleukin-6-dependent gene expression profiles in multiple myeloma INA-6 cells reveal a Bcl-2 family-independent survival pathway closely associated with Stat3 activation. Blood 103:242*.)*

Pancreas carcinoma
(Coppola, D. 2000. Molecular prognostic markers in pancreatic cancer. Cancer Control 7:421*.*
Scholz, A., S. Heinze, K. M. Detjen, M. Peters, M. Welzel, P. Hauff, M. Schirner, B. Wiedenmann, and S. Rosewicz. 2003. Activated signal transducer and activator of transcription 3 (STAT3) supports the malignant phenotype of human pancreatic cancer. Gastroenterology 125:891*.)*

Prostate carcinoma
(Torres-Roca, J. F., M. DeSilvio, L. B. Mora, L. Y. Khor, E. Hammond, N. Ahmad, R. Jove, J. Forman, R. J. Lee, H. Sandler, and A. Pollack. 2007. Activated STAT3 as a correlate of distant metastasis in prostate cancer: a secondary analysis of Radiation Therapy Oncology Group 86-10. Urology 69:505*.)*

Thymic epithelial tumours
(Chang, K. C., M. H. Wu, D. Jones, F. F. Chen, and Y. L. Tseng. 2006. Activation of STAT3 in thymic epithelial tumours correlates with tumour type and clinical behaviour. J. Pathol. 210:224*.)*

In the following a list of inflammatory diseases and autoimmune diseases is provided with an involvement of hyper activated transcription factor Stat3 (references to publications documenting the link between the respective disease and Stat3 are provided in brackets):

Asthma
(Simeone-Penney, M. C., M. Severgnini, P. Tu, R. J. Homer, T. J. Mariani, L. Cohn, and A. R. Simon. 2007. Airway Epithelial STAT3 Is Required for Allergic Inflammation in a Murine Model of Asthma. J. Immunol. 178:6191*.)*

Autoimmune diabetes (insulin-dependent diabetes)
(Ishihara, K., and T. Hirano. 2002. IL-6 in autoimmune disease and chronic inflammatory proliferative disease. Cytokine Growth Factor Rev. 13:357*.)*

Colitis
(Atreya, R., J. Mudter, S. Finotto, J. Mullberg, T. Jostock, S. Wirtz, M. Schutz, B. Bartsch, M. Holtmann, C. Becker, D. Strand, J. Czaja, J. F. Schlaak, H. A. Lehr, F. Autschbach, G. Schurmann, N. Nishimoto, K. Yoshizaki, H. Ito, T. Kishimoto, P. R. Galle, S. Rose-John, and M. F. Neurath. 2000. Blockade of interleukin 6 trans signaling suppresses T-cell resistance against apoptosis in chronic intestinal inflammation: evidence in crohn disease and experimental colitis in vivo. Nat. Med. 6:583*.*
Atreya, R., and M. F. Neurath. 2005. Involvement of IL-6 in the pathogenesis of inflammatory bowel disease and colon cancer. Clin. Rev. Allergy Immunol. 28:187*.*)

Crohn disease (Morbus Crohn)
(Atreya, R., J. Mudter, S. Finotto, J. Mullberg, T. Jostock, S. Wirtz, M. Schutz, B. Bartsch, M. Holtmann, C. Becker, D. Strand, J. Czaja, J. F. Schlaak, H. A. Lehr, F. Autschbach, G. Schurmann, N. Nishimoto, K. Yoshizaki, H. Ito, T. Kishimoto, P. R. Galle, S. Rose-John, and M. F. Neurath. 2000. Blockade of interleukin 6 trans signaling suppresses T-cell resistance against apoptosis in chronic intestinal inflammation: evidence in crohn disease and experimental colitis in vivo. Nat. Med. 6:583.
Atreya, R., and M. F. Neurath. 2005. Involvement of IL-6 in the pathogenesis of inflammatory bowel disease and colon cancer. Clin. Rev. Allergy Immunol. 28:187*.*)

Glomerulonephritis
(Takahashi, T., H. Abe, H. Arai, T. Matsubara, K. Nagai, M. Matsuura, N. lehara, M. Yokode, S. Nishikawa, T. Kita, and T. Doi. 2005. Activation of STAT3/Smad1 is a key signaling pathway for progression to glomerulosclerosis in experimental glomerulonephritis. J. Biol. Chem. 280:7100*.)*

Inflammatory bowel disease
(Atreya, R., J. Mudter, S. Finotto, J. Mullberg, T. Jostock, S. Wirtz, M. Schutz, B. Bartsch, M. Holtmann, C. Becker, D. Strand, J. Czaja, J. F. Schlaak, H. A. Lehr, F. Autschbach, G. Schurmann, N. Nishimoto, K. Yoshizaki, H. Ito, T. Kishimoto, P. R. Galle, S. Rose-John, and M. F. Neurath. 2000. Blockade of interleukin 6 trans signaling suppresses T-cell resistance against apoptosis in chronic intestinal inflammation: evidence in crohn disease and experimental colitis in vivo. Nat. Med. 6:583.
Atreya, R., and M. F. Neurath. 2005. Involvement of IL-6 in the pathogenesis of inflammatory bowel disease and colon cancer. Clin. Rev. Allergy Immunol. 28:187*.*)

Multiple sclerosis
(Frisullo, G., F. Angelucci, M. Caggiula, V. Nociti, R. lorio, A. K. Patanella, C. Sancricca, M. Mirabella, P. A. Tonali, and A. P. Batocchi. 2006. pSTAT1, pSTAT3, and T-bet expression in peripheral blood mononuclear cells from relapsing-remitting multiple sclerosis patients correlates with disease activity. J. Neurosci. Res. 84:1027*.)*

Osteoporosis
(Ishihara, K., and T. Hirano. 2002. IL-6 in autoimmune disease and chronic inflammatory proliferative disease. Cytokine Growth Factor Rev. 13:357.)

Pancreatitis
(Chao, K. C., K. F. Chao, C. C. Chuang, and S. H. Liu. 2006. Blockade of interleukin 6 accelerates acinar cell apoptosis and attenuates experimental acute pancreatitis in vivo. Br J Surg 93:332.)

Psoriasis
(Ishihara, K., and T. Hirano. 2002. IL-6 in autoimmune disease and chronic inflammatory proliferative disease. Cytokine Growth Factor Rev. 13:357*.*
Sano, S., K. S. Chan, S. Carbajal, J. Clifford, M. Peavey, K. Kiguchi, S. Itami, B. J. Nickoloff, and J. DiGiovanni. 2005. Stat3 links activated keratinocytes and immunocytes required for development of psoriasis in a novel transgenic mouse model. Nat. Med. 11:43*.)*

Rheumatoid arthritis
(Ishihara, K., and T. Hirano. 2002. IL-6 in autoimmune disease and chronic inflammatory proliferative disease. Cytokine Growth Factor Rev. 13:357.)

Systemic lupus erythematosus
(Liang, B., D. B. Gardner, D. E. Griswold, P. J. Bugelski, and X. Y. Song. 2006. Anti-interleukin-6 monoclonal antibody inhibits autoimmune responses in a murine model of systemic lupus erythematosus. Immunology 119:296*.*)

Systemic-onset juvenile chronic arthritis (JCA)
(Ishihara, K., and T. Hirano. 2002. IL-6 in autoimmune disease and chronic inflammatory proliferative disease. Cytokine Growth Factor Rev. 13:357.)

It is well-known that with the advent of molecular medicine diagnosis and classification of diseases is no longer exclusively based on established clinical parameters, but instead and to an increasing extent involves the determination of the molecular status of the diseased cells or tissues. Such determination may involve expression profiling at the mRNA and/or protein expression level and/or the determination of the activation status of one or more biomolecules, wherein posttranslational modifications may be indicative of the activation status.

For the purposes of the present invention, and in particular for determining whether a disease is a disease with an involvement of Stat3 activation for aetiology and/or progression, it is envisaged to determine the phosphorylation status of Stat3, preferably the phosphorylation status of tyrosine residues, wherein a higher degree of phosphorylation is indicative of the activated form of Stat3. More preferred, said phosphorylation status is the phosphorylation status of tyrosine residue 705 in the sequence of human Stat3 as shown in SEQ ID NO: 7 or of a corresponding tyrosine residue in Stat3 from another species. The term "corresponding" refers to one or more residues, in this context a tyrosine residue, which align in pairwise or multiple sequence alignments of Stat3 protein sequences from different species. Methods for sequence alignment and comparison are described herein above. Suitable means for determining the phosphorylation status of a protein or polypeptide are known in the art and include the use of antibodies, derivatives or fragments thereof which recognise, preferably specifically recognise a phosphorylated tyrosine in the respective context (e.g. anti-phosphoStat3 antibody no. 9131 from Cell Signaling Technology (Beverly, MA); for usage of this antibody see e.g. reference 11).

Alternatively or in addition, the expression level of Stat3 target genes, including that of miR-21, may be determined in order to assess whether a disease is Stat3-dependent. As stated above, said expression level may be the mRNA and/or the protein expression level. Means for determining the expression level are known in the art and described herein. Accordingly, the steps of the method of diagnosing according to the invention may be applied in order to determine whether a disease of condition a patient suffers from is a Stat3-dependent disease, wherein a higher amount as compared to a sample from a healthy subject of said nucleic acid the expression level of which is to be determined is indicative of said disease or condition being Stat3-dependent.

The present invention also relates to a method of identifying a compound suitable for the treatment of cancer, inflammatory diseases, autoimmune diseases, or a disease with an involvement of Stat3 activation for aetiology and/or progression; or suitable as a lead compound for the development of such compound suitable for treatment; the method comprising the steps of (a) bringing into contact Stat3; a test compound; and a nucleic acid comprising a double-stranded part, said part comprising the sequence of any one of SEQ ID NOs 1 to 5; (b) determining (i) the amount of complex formed between Stat3 and said nucleic acid; (ii) modifications of said nucleic acid and/or of histones, if any, bound thereto, said modifications being indicative of silencing; and/or (iii) the amount of transcript of a gene being under the control of said nucleic acid; in the presence of said test compound; and (c) comparing (i) the amount determined in step (b)(i) with the amount of said complex formed in absence of said test compound; (ii) modifications determined in step (b)(ii) with the modifications in absence of said test compound; and/or (iii) the amount determined in step (b)(iii) with the amount of transcript of said gene in absence of said test compound; wherein a lower amount determined in step (b)(i); more or different modifications determined in step (b)(ii); and/or a lower amount of transcript determined in step (b)(iii) is/are indicative of a compound suitable for treatment or as a lead compound.

This embodiment provides a screening assay for the identification of compounds capable of interfering with the interaction of Stat3 with the Stat3 binding region upstream of the coding region of the miR-21 gene. The screening method may employ any one of three readout schemes (i) to (iii), which may also be used in combination.

To explain further, the efficacy of compounds to block or reduce expression of genes under the control of said nucleic acid are analysed using cellular models. As stated above, said genes include the miR-21 gene. Compounds are be administered to the cells by delivery methods that are optimised for the cell system chosen, e.g. by electroporation, lipofection, or other standard transfection techniques.

As an example, the human cell line LNCaP (ATCC-Number: CRL-1740) is used as a model for prostate carcinoma. In these cells, miR-21 expression is low but can be induced by Stat3-activation agents like interkeukin 6. Test compounds will be delivered, preferably transfected by electroporation into these cells. For transfection, the amaxa biosystems (Cologne, Germany) Nucleofactor technology is used. Successful blockade of Stat3-dependent miR-21 gene transcription will be monitored by the three criteria (i) to (iii) either alone or in combination. Exemplary means and methods for assessing these criteria are provided in the Examples enclosed herewith.

The term "lead compound" is well-known in the art and includes compounds identified in a screen, the optimization of the pharmacological properties of which is still considered necessary or desirable. Methods for the optimization of the pharmacological properties of lead compounds are known in the art and comprise a method of modifying a compound identified as a lead compound to achieve: (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmacokinetic parameters (resorption, distribution, metabolism and excretion), and/or (vii) modified physico-chemical parameters (solubility, hygroscopicity, colour, taste, odor, stability, state), and/or (viii) improved general specificity, organ/tissue specificity, and/or (ix) optimized application form and route by (i) esterification of carboxyl groups, or (ii) esterification of hydroxyl groups with carboxylic acids, or (iii) esterification of hydroxyl groups to, e.g. phosphates, pyrophosphates or sulfates or hemi-succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogs, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiffs bases, oximes, acetales, ketales, enolesters, oxazolidines, thiazolidines or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

Said "bringing into contact" refers to bringing into contact under conditions permitting the formation of a complex between Stat3 and the recited nucleic acid when a test compound is absent. Preferably, the method of identifying a compound according to the invention is implemented as a cellular screen. The cellular environment ensures that said conditions are such that formation of said complex can occur.

Said nucleic acid comprising a double-stranded part, said part comprising the sequence of any one of SEQ ID NOs: 1 to 5 may be the upstream region of a gene encoding miR-21 or a homologue thereof, wherein the region includes the enhancer where Stat3 binds. Alternatively, said nucleic acid may also consist of or comprise any one of the sequences of SEQ ID NOs: 1 to 5 operatively linked to a gene encoding miR-21 or any gene different from miR-21, for example a reporter gene. Alternatively, said nucleic acid may not comprise a coding region. The latter applies to those embodiments which do not involve determining the amount of transcript of a gene. It is furthermore understood that the sequence of any one of SEQ ID NOs: 1 to 5 is located on one strand of said double-stranded part.

The term "test compound" is not limited to any particular compound. It comprises small molecules such as small organic molecules, preferably with a molecules weight below 2000, more preferred below 1000, and yet more preferred below 500. Also comprised by the term "test compound" are polynucleotides and oligonucleotides as well as analogs and derivatives thereof. Further examples of test compounds are peptides. The term "test compound" also comprises compounds which are comprised in an extract obtained from natural source such as a plant extract. Extracts are frequently tested in high-throughput screens, generally in addition to the testing of pure or substantially pure compounds of natural or non-natural origin.

Test compounds which are suitable for treatment or as a lead compound are those which interfere with the complex formation between Stat3 and said nucleic acid, thereby giving rise to a lesser amount of complex; test compounds which trigger the silencing mechanisms of the cell and/or compounds which reduce the amount of transcript read from said gene.

For the purposes of the method of identifying a compound according the present invention, Stat3 may be used in the form of a fusion protein comprising the sequence of Stat3. Furthermore, it is envisaged to use the binding domain of Stat3 as opposed to the Stat3 sequence in its entirety, wherein the term "binding domain" refers to that subsequence of Stat3 which, upon folding, is capable of binding to those nucleic acids full-length Stat3 is binding to. An example of such a nucleic acid is the above recited nucleic acid comprising a double-stranded part, said part comprising the sequence of any one of SEQ ID NOs: 1 to 5. Said binding domain may be part of a fusion protein.

In a preferred embodiment of the method of identifying a compound according to the invention, said double-stranded part is double-stranded DNA and said modifications are methylation of said DNA or acetylation, methylation, or phosphorylation of histones bound to said DNA, if any histones are bound to said DNA.

In a further preferred embodiment of the method of identifying a compound according to the invention, said method further comprises the steps of: (d) bringing into contact Stat3; said test compound; and a nucleic acid comprising a double-stranded part, said part comprising the sequence of the Stat3-binding region of a gene selected from the group consisting of haptoglobin, ICAM1, SERPINA3, SOCS3 and BCL3; (e) determining (i) the amount of complex formed between Stat3 and said nucleic acid defined in (d); (ii) modifications of said nucleic acid defined in (d) and/or of histones, if any, bound thereto, said modifications being indicative of silencing; and/or (iii) the amount of transcript of a gene being under the control of said nucleic acid defined in (d); in the presence of said test compound; and (f) comparing (i) the amount determined in step (e)(i) with the amount of said complex formed in absence of said test compound; (ii) modifications determined in step (e)(ii) with the modifications in absence of said test compound; and/or (iii) the amount determined in step (e)(iii) with the amount of transcript of said gene in absence of said test compound; wherein equal amounts of the complex as defined in (e)(i), identical modifications or equal numbers of modifications, said modifications being defined in (e)(ii), and/or equal amounts of transcript as defined in (e)(iii) are indicative of a test compound not interfering with the interaction of Stat3 with said nucleic acid defined in (d).

Preferably, said sequence of the Stat3-binding region is selected from SEQ ID NO: 8 to 17).

The purpose of this embodiment is to provide a comparison between the capability of a test compound to interfere with the interaction between Stat3 and the enhancer region of the miR-21 gene on the one side and the capability of said test compound to interfere with the interaction between Stat3 and the Stat3 binding region on one or more Stat3 target genes different from the gene encoding miR-21. A compound which interferes with the interaction between Stat3 and the enhancer in a miR-21 gene, but does not interfere with Stat3 binding to the Stat3 binding regions in one or more of the above-defined genes, is particularly of interest since it interferes with Stat3 signaling only to the extent miR-21 or homologues thereof as well as downstream effectors thereof are involved.

The present invention also provides the use of a nucleic acid, analog or derivative thereof, wherein said nucleic acid, analog or derivative thereof consists of or comprises a base sequence, said base sequence (a) being complementary to the full length of the sequence of SEQ ID NO: 6; (b) being capable of hybridising under stringent conditions with the sequence of SEQ ID NO: 6; or (c) having at least 80% sequence identity to the sequence of (a); for the manufacture of a pharmaceutical composition for the treatment of inflammatory diseases, autoimmune diseases, acute myeloid leukaemia, anaplastic large cell lymphoma, cholangiocarcinoma, colon carcinoma (colorectal cancer), head and neck cancer, hepatocellular carcinoma, Hodgkin lymphoma, lung cancer, melanoma, multiple myeloma, pancreas carcinoma, prostate carcinoma and thymic epithelial tumours, or a disease with an involvement of Stat3 activation for aetiology and/or progression.
The above-recited nucleic acid, analog or derivative thereof is directed to the transcript of the miR-21 gene and homologs thereof as opposed to the upstream enhancer region comprised in a gene encoding miR-21.

Preferably, said sequence identity is 85%, 90% or 95%, more preferred 96%, 97%, 98% or 99%.

The term "hybridizes/hybridizing" as used herein refers to a pairing of a nucleic acid to a (partially) complementary strand of this nucleic acid which thereby form a hybrid.

It is well known in the art how to perform hybridization experiments with nucleic acid molecules. Correspondingly, the person skilled in the art knows what hybridization conditions s/he has to use to allow for a successful hybridization in accordance with the present invention. The establishing of suitable hybridization conditions is referred to in standard text books such as Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985). In one preferred embodiment, the hybridization is effected is under stringent conditions.

"Stringent hybridization conditions" refers to conditions which comprise, e.g. an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing the filters in 0.1 x SSC at about 65°C. Said conditions for hybridization are also known by a person skilled in the art as "highly stringent conditions for hybridization".

Also contemplated are nucleic acid molecules that hybridize to the respective target sequences according to the invention at lower stringency hybridization conditions ("low stringency conditions for hybridization"). Changes in the stringency of hybridization and signal detection are primarily accomplished through the manipulation of formamide concentration (lower percentages of formamide result in lowered stringency), salt conditions, or temperature. For example, lower stringency conditions include an overnight incubation at 37°C in a solution comprising 6X SSPE (20X SSPE = 3M NaCl; 0.2M NaH₂PO4; 0.02M EDTA, pH 7.4), 0.5% SDS, 30% formamide, 100 µg/ml salmon sperm blocking DNA; followed by washes at 50°C with 1X SSPE, 0.1% SDS. In addition, to achieve an even lower stringency, washes performed following stringent hybridization can be done at higher salt concentrations (e.g. 5X SSC). It is of note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Such modifications can generally be effected by the skilled person without further ado. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed).

Also provided herein is an in vitro or ex vivo method of diagnosing (a) a disease or condition selected from the group consisting of inflammatory diseases, autoimmune diseases, multiple myeloma, prostate carcinoma, and diseases with an involvement of Stat3 activation for aetiology and/or progression; or (b) subtypes of such disease exhibiting different prognosis or treatment responsiveness, the method comprising determining in a sample obtained from a subject the amount of nucleic acid consisting of or comprising (i) the sequence of SEQ ID NO: 6; or (ii) a sequence having at least 80% sequence identity to the sequence of SEQ ID NO: 6 over the entire length of the sequence of SEQ ID NO: 6, wherein a higher amount as compared to a sample obtained from a healthy subject, also referred to as "over-expression", is indicative of (a) said disease or condition; or (b) of worse prognosis or treatment responsiveness. Preferably, said nucleic acid is a microRNA.

Preferably, said sequence identity is 85%, 90% or 95%, more preferred 96%, 97%, 98% or 99%.

The term "prognosis" refers to a prediction of the further course of said disease or condition, wherein a worse prognosis includes an aggravation of said disease or condition and/or a possibly lethal outcome. The lethal outcome may be expected to occur at an earlier point in time as compared to a patient with good prognosis. In case of malignant diseases, prognosis is correlated with the incidence of metastases.

The term "treatment responsiveness" refers to the manner and extent a patient presents improvement of the symptoms of a disease or condition upon treatment, e.g. with drugs. Worse responsiveness refers to less or no improvement as compared to a patient with exhibiting good responsiveness.

Determining the amount of said nucleic acid, i.e. the expression level thereof, can be performed by methods well known in the art. The term "over-expression" denotes an expression level of said nucleic acid, preferably microRNA, which is elevated in comparison to normal expression. The term "normal expression" refers to a reference expression level determined in one or more samples from healthy individuals. These samples are preferably from healthy tissue corresponding to the tissue affected by the disease or condition under consideration. Samples may be drawn from a mixed population, from a fraction of the population, wherein the population has previously been stratified according to one or more parameters, or from healthy regions of the tissue affected by the disease or condition from the same patient. Statistical methods known in the art may be used in order to assign significance values and confidence intervals to the measured expression and over-expression data. For example, miR-21 expression levels will be measured in tissue or cell samples obtained from patients and correlated with the patients' data with respect to disease scores, treatment effects, or survival rates. miR-21 expression will be analysed as described below (Example 2) using real-time PCR.

Preferably, said nucleic acid, the amount of which is to be determined, is a microRNA.

Alternatively, the expression level of the primary transcript of the miR-21 gene may be determined. The term "primary transcript" refers to the immediate product of transcription as opposed to the processed microRNA the sequence of which is set forth in SEQ ID NO: 6.

Methods for the determination of such RNA expression levels are known in the art and comprise Real Time PCR, Northern blotting and hybridization on microarrays or DNA chips equipped with one or more probes or probe sets specific for transcripts consisting of or comprising the sequence of SEQ ID NO: 6 or homologues as defined above.

Alternatively or in addition, the expression level of one or more molecules acting downstream of miR-21 may be determined. The term "downstream" refers to molecules the activity and/or expression of which is influenced by activity and/or expression of miR-21.

In this regard, the expression level to be determined may be or may include the protein expression level. The skilled person is aware of methods for the quantitation of proteins. Amounts of purified protein in solution can be determined by physical methods, e.g. photometry. Methods of quantifying a particular protein in a mixture rely on specific binding, e.g of antibodies. Specific detection and quantitation methods exploiting the specificity of antibodies comprise immunohistochemistry (*in situ)* and surface plasmon resonance. Western blotting combines separation of a mixture of proteins by electrophoresis and specific detection with antibodies.

In a preferred embodiment of the use or the method of the invention, said inflammatory disease or autoimmune disease is selected from the group consisting of asthma, autoimmune diabetes (insulin-dependent diabetes), colitis, crohn disease (morbus Crohn), glomerulonephritis, inflammatory bowel disease, multiple sclerosis, osteoporosis, pancreatitis, psoriasis, rheumatoid arthritis, systemic lupus erythematosus and systemic-onset juvenile chronic arthritis (JCA).

In another preferred embodiment of the use of the method according to the invention, said disease with an involvement of Stat3 activation for aetiology and/or progression is a cancer with an involvement of Stat3 activation for aetiology and/or progression.
In a further preferred embodiment of the use or the method of the preceding embodiment, said cancer with an involvement of Stat3 activation for aetiology and/or progression is selected from the group consisting of Stat3-dependent acute myeloid leukaemia, Stat3-dependent anaplastic large cell lymphoma, Stat3-dependent cholangiocarcinoma, Stat3-dependent colon carcinoma (colorectal cancer), Stat3-dependent glioblastoma, Stat3-dependent head and neck cancer, Stat3-dependent hepatocellular carcinoma, Stat3-dependent Hodgkin lymphoma, Stat3-dependent lung cancer, Stat3-dependent mammary carcinoma, Stat3-dependent melanoma, Stat3-dependent multiple myeloma, Stat3-dependent pancreas carcinoma, Stat3-dependent prostate carcinoma and Stat3-dependent thymic epithelial tumours.

In preferred embodiments of the compounds of the invention, the pharmaceutical composition of the invention or the use of the invention, said analog or derivative is selected from the group consisting of peptide nucleic acids, phosphorothioate nucleic acids, phosphoramidate nucleic acids, 2'-O-methoxyethyl ribonucleic acids, morpholino nucleic acids, hexitol nucleic acids, locked nucleic acids, chimeras thereof and chimeras of oligonucleotides or polynucleotides and any one of peptide nucleic acids, phosphorothioate nucleic acids, phosphoramidate nucleic acids, 2'-O-methoxyethyl ribonucleic acids, morpholino nucleic acids, hexitol nucleic acids and locked nucleic acids.

In other words, further included are polynucleotide- or nucleic acid-mimicking molecules known in the art such as synthetic or semisynthetic derivatives of DNA or RNA and mixed polymers. They may contain additional non-natural or derivatized nucleotide bases, as will be readily appreciated by those skilled in the art. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see, for example, (49)). LNA is an RNA derivative in which the ribose ring is constrained by a methylene linkage between the 2'-oxygen and the 4'-carbon.

For the purposes of the present invention, a peptide nucleic acid (PNA) is a polyamide type of DNA analog. The monomeric units for the corresponding derivatives of adenine, guanine, thymine and cytosine are available commercially (for example from Perceptive Biosystems). PNA is a synthetic DNA-mimic with an amide backbone in place of the sugar-phosphate backbone of DNA or RNA. As a consequence, certain components of DNA, such as phosphorus, phosphorus oxides, or deoxyribose derivatives, are not present in PNAs. As disclosed by Nielsen et al. (50) and Egholm et al. (51), PNAs bind specifically and tightly to complementary DNA strands and are not degraded by nucleases. Furthermore, they are stable under acidic conditions and resistant to proteases (52). Their electrostatically neutral backbone increases the binding strength to complementary DNA as compared to the stability of the corresponding DNA-DNA duplex (53, 54). In fact, PNA binds more strongly to DNA than DNA itself does. This is probably because there is no electrostatic repulsion between the two strands, and also the polyamide backbone is more flexible. Because of this, PNA/DNA duplexes bind under a wider range of stringency conditions than DNA/DNA duplexes, making it easier to perform multiplex hybridization. Smaller probes can be used than with DNA due to the strong binding. In addition, it is more likely that single base mismatches can be determined with PNA/DNA hybridization because a single mismatch in a PNA/DNA 15-mer lowers the melting point (Tₘ) by 8°-20° C, vs. 4°-16° C for the DNA/DNA 15-mer duplex. Thereby discrimination between perfect matches and mismatches is improved. For its uncharged nature, PNA also permits the hybridisation of DNA samples at low salt or no-salt conditions, since no interstrand repulsion as between two negatively charged DNA strands needs to be counteracted. As a consequence, the target DNA has fewer secondary structures under hybridisation conditions and is more accessible to probe molecules.

The term "derivatives" in conjunction with the above described peptide nucleic acids, phosphorothioate nucleic acids, phosphoramidate nucleic acids, 2'-O-methoxyethyl ribonucleic acids, morpholino nucleic acids, hexitol nucleic acids, locked nucleic acids and the above described chimeras relates to molecules wherein these molecules comprise one or more further groups or substituents different from peptide nucleic acids, phosphorothioate nucleic acids, phosphoramidate nucleic acids, 2'-O-methoxyethyl ribonucleic acids, morpholino nucleic acids, hexitol nucleic acids, locked nucleic acids and the above described chimeras as such. All groups or substituents known in the art and used for the synthesis of these molecules, such as protection groups, and/or for applications involving these molecules, such as labels and (cleavable) linkers are envisaged.

In those embodiments where the nucleic acid or the polynucleotide comprises (rather than consists of) the recited sequence, additional nucleotides or analogs thereof extend over the specific sequence either on the 5' end or the 3' end or both. Those additional oligo- or polynucleotides or analogs thereof may be of heterologous or homologous nature and may comprise stretches of about 50 to 500 nucleotides although higher or lower values are not excluded. In the case of homologous sequences, those embodiments do not include complete genomes and are generally confined to about 1500 additional nucleotides at the 5' and/or the 3' end. Additional heterologous sequences may include heterologous promoters which are operatively linked to the sequences of the invention.

In a further preferred embodiment of the compound of the invention the pharmaceutical composition of the invention or the use of the invention, said polynucleotide, nucleic acid, analog or derivative thereof is an antisense molecule, an siRNA, or a ribozyme, comprising said base sequence.

The term "antisense molecule" or "antisense construct" is known in the art and refers to a nucleic acid which is complementary to a target nucleic acid. An antisense molecule according to the invention is capable of interacting with, more specifically hybridizing with the target nucleic acid. By formation of the hybrid, transcription of the target gene(s) and/or translation of the target mRNA is reduced or blocked. Standard methods relating to antisense technology have been described (see, e.g., Melani et al. (55)).

Small interfering RNA (siRNA), sometimes known as short interfering RNA or silencing RNA, are a class of generally 20-25 nucleotide-long double-stranded RNA molecules that play a variety of roles in biology. Most notably, siRNA is involved in the RNA interference (RNAi) pathway where the siRNA interferes with the expression of a specific gene (see, e.g., (56, 57)). SiRNAs have a well defined structure, generally a short (usually 21-nt) double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. SiRNAs can also be exogenously (artificially) introduced into cells by various transfection methods to bring about the specific knockdown of a gene of interest. In this context, other structures than those described above are also envisaged, provided they are capable of interfering with gene expression. Essentially any gene of which the sequence is known can thus be targeted based on sequence complementarity with an appropriately tailored siRNA. This has made siRNAs an important tool for gene function and drug target validation studies as well as for therapeutic intervention which is envisaged here.

Ribozymes are enzymes consisting of or comprising a catalytically active ribonucleic acid which in turn comprises a sequence complementary to a sequence in the target nucleic acid. Ribozymes specifically cleave the target nucleic acid, such as the (pre)-mRNA of a gene, the consequence being reduction or repression of expression. The techniques underlying said repression of expression are well known in the art (see, e.g., EP-B1 0 291 533, EP-A1 0 321 201, EP-A2 0 360 257). Selection of appropriate target sites and corresponding ribozymes can be done as described for example in Steinecke et al. (58).

In a further preferred embodiment of the pharmaceutical composition of the invention the use or the method of the invention, said Stat3 is human Stat3, the sequence of which is shown in SEQ ID NO: 7.

The Figures show:

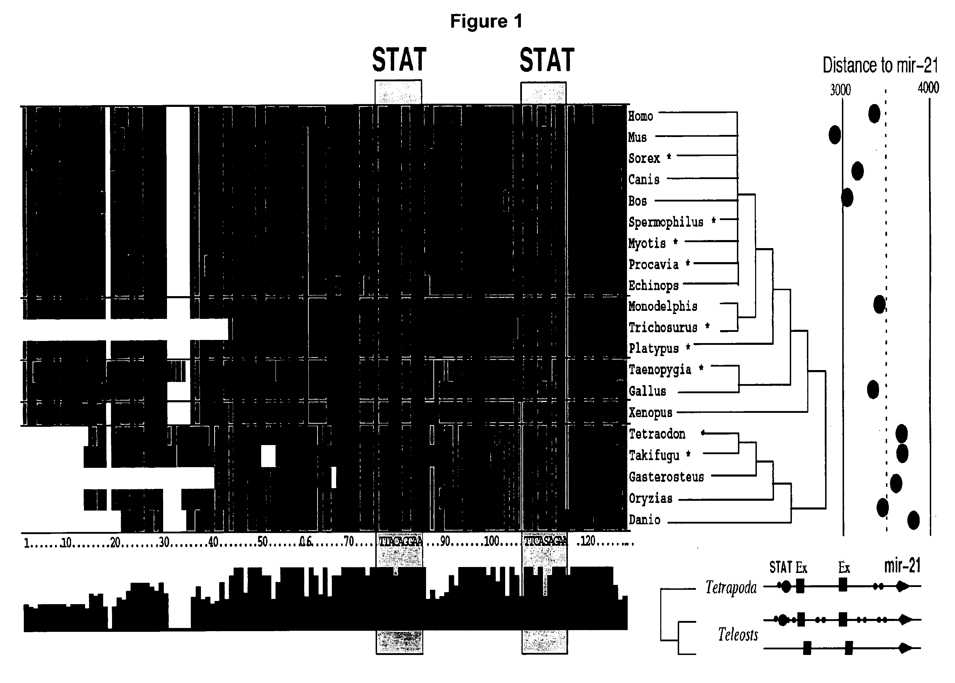

**Figure 1****: Alignment of the conserved regulatory region including two Stat3 binding sites upstream of the miR-21 gene.** The correlation of predicted Stat3 binding sites with 4000 bp regions upstream of known miRNA genes was analyzed using pwmatch, a re-implementation of the MATCH scoring algorithm published by Kel et al. (59). Evolutionarily conserved putative Stat3 binding sites were identified by the PhastCons track (60) using a consensus matrix based upon previously determined Stat3 binding sites (61, 62). For the miR-21 gene, the available genomic sequences of vertebrate organisms were searched for conserved non-coding sequences between 4 kb upstream and 2 kb downstream of the miRNA coding sequence with tracker (63). ***Left:*** 130-bp regions containing two predicted Stat3 binding sites upstream of the miR-21 genes of various vertebrate species are aligned. T, G, C, and A nucleotides are colored green, orange, blue and red, respectively. Stat3 sites are highlighted by yellow boxes. Sequences from organisms where no genomic sequence was available (as marked with a star) were obtained from the Ensembl trace repository. ***Upper right:*** The distance (in bp) between the miR-21 sequence and the Stat3 binding sites is given for all organisms where genomic data were available. ***Lower right:*** Conserved clusters in the miR-21 gene region. Teleost species harbor two copies of miR-21 (red arrows) but only one of them contains the conserved upstream regulatory region (STAT). Two of the detected footprints are conserved in vertebrates and both gene copies of teleosts (cluster 31 and 62 of Table S1). These conserved regions match the exons of TMEM49 (Ex). Other conserved clusters (small circles) are either tetrapod specific or are shared by the copy of teleost miR-21 that contains the Stat3 binding sites.

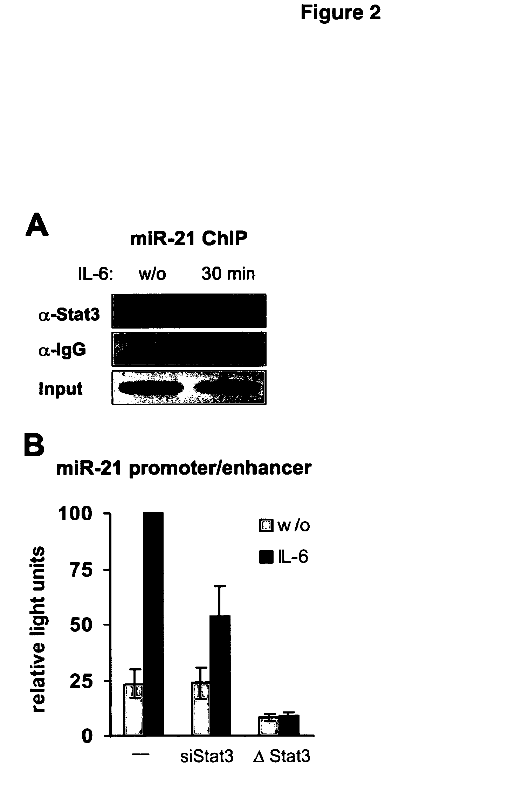

**Figure 2****: The miR-21 upstream regulatory region is a functional Stat3-controlled enhancer. (A)** XG-1 cells were deprived from IL-6 for 72 hours or restimulated for 30 min and subjected to a CHIP assay using anti-Stat3 or control serum. Co-immunoprecipitated DNA was amplified by PCR with primers specific for the miR-21 upstream enhancer. **(B)** Reporter gene assays were performed in HepG2 cells transfected with the miR-21 promoter/enhancer either alone (-) or in the presence of an siStat3-expressing plasmid (siStat3) or, with the promoter/enhancer lacking the two Stat3 sites (ΔStat3). Values represent mean luciferase activity ± standard deviation of 3 independent experiments relative to the unmanipulated sample treated with IL-6.

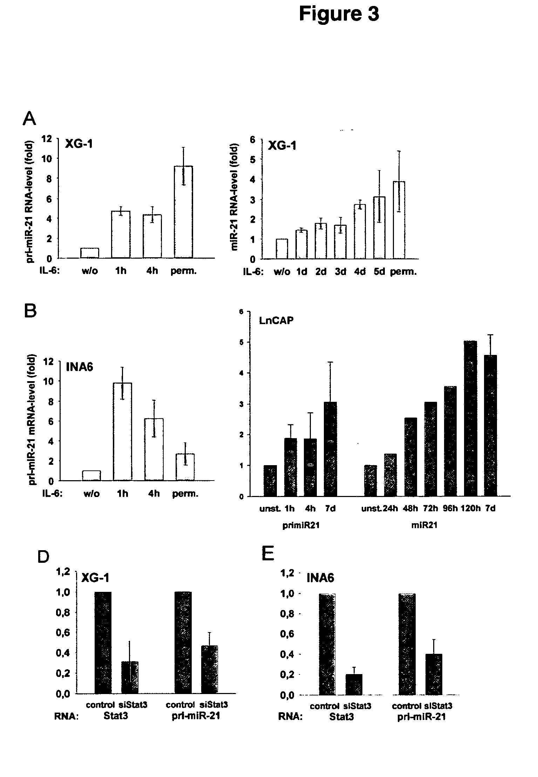

**Figure 3** : **IL-6 induces miR-21 expression via Stat3 activation. (A)** XG-1 and INA-6 cells were either restimulated with IL-6 for the times indicated after cytokine-withdrawal for 72 and 12 h, respectively, or continuously cultured with IL-6 (perm.). LNCaP and HepG2 cells were stimulated with IL-6 as indicated. RNA was extracted and pri-miR-21 expression measured by real-time PCR. Levels of mature miR-21 were quantified by stem-loop reverse transcription followed by real-time PCR (64). Values obtained for cells deprived of IL-6 were set to 1. **(B)** XG-1 and INA-6 cells were transiently transfected with an expression plasmid for an small-hairpin RNA silencing Stat3 (siStat3) or a scrambled sequence RNA (control) together with a vector encoding enhanced green fluorescence protein. After 48 hours, green fluorescent cells were sorted, RNA was extracted, and Stat3 and pri-miR-21 RNA levels were determined by real-time PCR. Values for the control samples were taken as reference. All data represent mean values ± standard deviation of three independent experiments.
**Figure 4****: Sequence alignments of the human Stat3-dependent enhancer of the miR-21 gene with the corresponding sequences from chimpanzee, dog, mouse, chicken and frog.** The high degree of conservation, in particular among the mammalian species is apparent.

The following examples illustrate the invention but should not be construed as being limiting.

### Example 1

To investigate the possibility that Stat3 is involved in the regulation of microRNA genes, we analyzed the genomic distribution of predicted conserved Stat3 binding sites and their correlation with known microRNAs by phylogenetic footprinting. We found that 40 out of 397 microRNAs listed in the MicroRNA Registry 8.1 (65, 66) are associated with putative Stat3 binding sites identified as being evolutionarily conserved. Among them, miR-21 has been described as an oncogenic miRNA ("oncomir") exhibiting antiapoptotic activity in glioblastoma and cholangiocarcinoma cells (34-36). We therefore undertook closer inspection of the upstream region of the miR-21 gene in several vertebrate species to determine its evolutionary conservation. The putative regulatory region is located within an intron of the transmembrane protein 49 (TMEM49) gene and contains two consensus Stat3 binding sites, TTACAGGAA and TTCTGAGAA. For organisms where genomic sequence was available we investigated all conserved non-coding sequences from 4 kb upstream to 2 kb downstream of the miR-21 coding sequence. It is evident from Fig. 1 that the Stat3 sites and the surrounding region are highly conserved in vertebrates. Altogether the conserved sequence block spans about 300 bp (Table S1). Unless we see dramatic assembly errors, the genomic data suggest that miR-21 and its associated Stat3 binding sites are functionally independent of TMEM49, since the linkage of these entities appears to be broken up in some teleosts (see supplementary material). The analysis of the regulatory regions shows a stable distance of 3 to 3.8 kb between miR-21 and the Stat3 binding sites throughout all vertebrates. This is astonishing as the intervening coding sequences have lost their integrity and the length of such sequences normally correlates with the genome size of organisms (67). Teleost genomes are significantly smaller than the genomes of tetrapod species, and the size of a coding gene immediately upstream of miR-21 ranges from 140 kb in tetrapods to 5.5 kb in teleosts. Therefore, this observation strongly suggests that the stable distance between miR-21 and the Stat3 sites is of functional relevance.

To study whether Stat3 is indeed recruited to the miR-21 upstream region, we subjected IL-6-starved and restimulated XG-1 human myeloma cells to chromatin immunoprecipitation (ChIP). Stat3 binding to the upstream region increased by approximately one order of magnitude upon IL-6 treatment (Fig. 2A). Furthermore, a luciferase reporter assay verified the IL-6-responsiveness of a human genomic DNA fragment extending from -1120 to +25 base pairs relative to the miR-21 transcription start site (68) in HepG2 hepatocellular carcinoma cells (Fig. 2B). IL-6-induced miR-21 promoter activity was inhibited by coexpressing a Stat3-specific small hairpin RNA, and was entirely abolished by eliminating the Stat3 binding motifs by site-directed mutagenesis (Fig. 2C). These data demonstrate the existence of a functional Stat3-regulated enhancer upstream of the miR-21 gene.

We next examined whether IL-6-induced Stat3 activation yields enhanced transcription of the miR-21 gene. For that purpose, myeloma cell lines XG-1 and INA-6 were withdrawn from IL-6, restimulated, and the primary miR-21 transcript (pri-miR-21) quantified by real-time PCR. IL-6 increased pri-miR-21 expression substantially in either cell line, albeit with different time courses (Fig. 3A). Maximal induction was reached in INA-6 cells already 1 h after IL-6 stimulation, whereas in XG-1 the expression continued to increase. In spite of the rapidly induced expression in the primary transcript level, mature miR-21 levels increased in XG-1 cells only modestly during the first days of IL-6 treatment and finally reached an about 4-fold elevation upon continuous stimulation. A comparably slow induction was also observed in LNCaP prostate carcinoma cells (Fig. 3A). Processing and/or turnover rates of miR-21, therefore, appear to be remarkably slow. This notion might also explain why in INA-6 cells, mature miR-21 expression increased only slightly (not shown) because in these cells cytokine withdrawal induces rapid apoptosis (11) and hence the 12-h withdrawal period likely does not suffice to efficiently reduce mature miR-21 levels. Likewise, in HepG2 hepatocellular carcinoma IL-6 treatment yielded a moderate pri-miR21 induction (Fig. 3A) and only marginally increased mature miR-21 levels (not shown).

To prove that miR-21 induction by IL-6 is mediated by Stat3, we silenced Stat3 expression in INA-6 and XG-1 cells by RNA interference. Stat3 knockdown resulted in an at least 60% inhibition of pri-miR-21 induction compared to control cells expressing a control small-hairpin RNA (Fig. 3B). Taken together, our data demonstrate that transcription of the miR-21 gene is under the control of IL-6 and requires the activation of Stat3.

MiR-21 is involved in the control of tumor cell apoptosis (34, 36). We therefore reasoned that miR-21 might be implicated in the Stat3-dependent survival of myeloma cells as well. If that assumption was true one would expect an ectopic miR-21 overexpression in these cells to at least partially by-pass their requirement for Stat3 activation. As a matter of fact, miR-21 overexpressed by transient transfection of INA-6 and XG-1 cells partially protected them from apoptosis induced by IL-6 withdrawal (Table 1, Fig. S1). We conclude that the thus established pathway leading to miR-21 induction represents an important contribution to the involvement of IL-6 and Stat3 in the pathogenesis of multiple myeloma and other malignancies. This view is further supported by the observation that in an intriguingly parallel fashion, inhibition of either, miR-21 or Stat3, induces apoptosis in glioblastoma cells (8, 34, 69) and sensitizes cholangiocarcinoma cells to drug- or TRAIL-induced apoptosis, respectively (36, 70). Taken together, these data provide strong evidence that induction of miR-21 plays a pivotal role for the antiapoptotic function of Stat3.

**Table 1: miR-21 promotes survival of INA-6 (and XG-1) myeloma cells***

| time | transfection | % viable cells | |
|---|---|---|---|
| | | w/o IL-6 | with IL-6 |
| 24 hours | control | 33.9 ± 2.5 | 49.1 ± 1.6 |
| | miR-21 | 51.2 ± 0.9 | 64.0 ± 1.3 |
| 48 hours | control | 26.0 ± 4.9 | 67.3 ± 2.0 |
| | miR-21 | 45.2 ± 5.6 | 79.7 ± 0.8 |

| | | | |
|---|---|---|---|
| *INA-6 cells cultivated in the presence of IL-6 were transiently transfected with a miR-21 expression vector or an empty control vector. An expression plasmid encoding enhanced green fluorescent protein was cotransfected. One day posttransfection, cell culture was continued either in the presence or absence of IL-6. After additional 24 or 48 h, cells were subjected to flow-cytometric Annexin V apoptosis assay. Green fluorescent, Annexin V- and 7-AAD-negative cells were considered viable. Data represent mean values ± standard deviation from three independent experiments. | | | |

Stat3 directly controls the expression of a number of antiapoptotic proteins on the transcriptional level, including survivin and the B-cell lymphoma-(Bcl)-2 family members Bcl-x and myeloid cell leukemia-1 (Mcl-1) (8, 70-72). In fact, Mcl-1 has been recognized as a critical survival factor for multiple myeloma (73, 74). The rapid transcriptional upregulation of such genes by Stat3, therefore, appears as an important fast-track survival signal for myeloma and presumably other tumor cells. However, recent data suggest that this fast regulatory route does not sufficiently explain the full antiapoptotic potential of Stat3, and hence the existence of additional Stat3-induced survival mechanisms was postulated (11, 75). As we demonstrate here, miR-21 induction by Stat3 represents a rather slow-acting but long-lasting survival route, which appears as an ideal complementation of the immediate induction of antiapoptotic factors. Considering the critical role of miR-21 in the apoptosis regulation of various tumor cells as reported here and elsewhere, this view infers a pivotal relevance of this miRNA for the oncogenic potential of Stat3. Accordingly, as part of a miRNA expression signature, miR-21 is overexpressed in numerous solid tumors including carcinomas and adenomas from breast, colon, lung, pancreas, prostate, and stomach. (32-34). In all these malignancies, Stat3 has either been reported to be constitutively activated or has been proven to be functionally involved in cell survival or growth.

Since miRNAs function as negative regulators of the stability and/or translation of specific target mRNAs, an important step to unravel the molecular mechanism of miR-21 action will be to identify its mRNA targets. Recently, miR-21 was reported to repress the expression of the tumor suppressor PTEN and thereby enhance the activity of the phosphatidyl inositol-3-kinase/AKT pathway (36). Yet, we did not detect an upregulation of PTEN expression or AKT phosphorylation in XG-1 cells in response to IL-6 (data not shown). Hence, the miR-21 targets involved in myeloma survival need to be identified by future research. Another open question regards the physiological role of miR-21. The strong phylogenetic conservation of the miRNA itself as well its Stat3-regulated enhancer suggests an important functional association of the two factors. In view of the critical involvement of Stat3 in embryonic development in fish (76, 77) it appears tempting to speculate that the primary role of miR-21 is closely associated with these developmental processes.

Stat3 has been widely recognized as a promising target for tumor therapy (9, 23, 24). However, the ubiquitous expression of the factor and its involvement in the control of numerous cellular processes in many cells and organs bears the risk of side reactions. Identified as a Stat3 target gene in this study, being upregulated in tumor cells, and critically involved in their survival, miR-21 therefore might prove a more selective target for the therapy of malignancies.

### Example 2

### Analysis of in situ-binding of Stat3 to particular chromatin regions by chromatin immunoprecipitation (ChlP)

*Chromatin immunoprecipitation (ChlP)* - The following ChlP is performed according to the protocol of ChlP assay kit (Upstate, Charlottesville, Virginia, USA). The cells analysed are serum-starved for 2 days. After activation of Stat3, usually by addition of interleukin 6 to the medium (10 ng/ml) for 30 min, cells (1.5 x10⁷) are cross-linked by addition of formaldehyde to the medium at a final concentration of 1% and incubated for 10 min at room temperature. After washing with ice-cold PBS, cells are harvested and resuspended in SDS lysis buffer. Chromatin is fragmented either by sonicatring the cell 4 times for 15 s at 60% amplitude using a Branson sonifier 250 (Branson, Danbury, CT) or enzymatically employing a mixture of endonucleases, resulting in DNA fragments with an average size of 500 bp. The lysates are then diluted to 2.2 ml in ChIP dilution buffer and precleared according to the protocol of Upstate Biology. Precleared lysates are incubated with anti-Stat3 antibodies overnight at 4°C. Antibody/target protein-complexes are collected by incubating with protein A agarose for 1 h at 4°C. After washing, each precipitate is eluted using elution buffer and cross-linking is reversed by heating to 65°C for 5 h. The samples are then treated with 2 ml proteinase K (10 mg/ml) for 1 h at 45°C, extracted with a phenol/chloroform/isoamyl alcohol mixture (25:24:1, v:v:v), and ethanol-precipitated in the presence of carrier glycogen. Pellets are resuspended in 30 ml of elution buffer. 5 ml of either IP-enriched DNA or unenriched DNA are amplified in 10-ml PCR reactions. PCR reactions were then analyzed on 1% agarose gels. Alternatively, precipitated DNA fragments are quantified by real-time PCR. To validate specificity and for normalisation, the following controls are carried out: for each experiment, target regions are amplified from the input cromatin (prior to immunoprecipitation), and immunoglobulin isotype controls are used (instead of specific Stat3 antibodies). Furthermore, a control chromatin region known to not bind Stat3 is also analysed.
PCR primers are chosen specific for the chromatin region under investigation and optimised by real-time PCR.

### Example 3

### Analysis of epigenetic changes in particular chromatin regions

### a) histone modifications (methylation and acetylation) are analysed by chromatin immunoprecipitation (ChIP):

The ChlP protocol is used as above, with the exception that instead of Stat3 antibodies, antibodies specifically recognising modified histones are applied.

### b) Analysis of DNA methylation of particalur chromatin regions:

Genomic DNA is isolated from the cells studied using the DNA isolation kit (Qiagen, Valencia, CA). Genomic DNA is digested with a restriction endonuclease, such as EcoRI, before chemical modification. Digested DNA (4 µg) in 40 µl is denatured with 0.3 mol/l NaOH for 15 minutes at 37° C. The denatured DNA is subsequently modified by 3.1 mol/l sodium bisulfite and 0.5 mmol/I hydroquinone in 480 µl under mineral oil for 16 hours at 55° C. Modified DNA is purified using the QlAquick DNA extraction kit according to the manufacturer (Qiagen). The DNA is eluted with 50 µl elution buffer [10 mmol/L Tris (pH 8.0)]. The modification is completed by 0.3 mol/l NaOH treatment for 15 minutes at 37° C. The modified DNA is precipitated by adding 3 mol/I ammonium acetate (pH 7.0) and 2 volumes of ethanol. After precipitation, DNA is resuspended in 50 µl water for PCR amplification. Two sequential PCRs are used to amplify the modified DNA fragments of interest. Modified DNA template (2 µl) is used in the first PCR, applying PCR primers that span the region of intersest. Nested primers are then used in a subsequent PCR. PCR conditions are optimised for the primer pairs used. The amplified DNA fragment of expected size are subjected to cloning using the pCR4TOPO TA cloning vector (Invitrogen), and at least 5 individual recombinant clones are sequenced.

### Example 4

### Analysis of miR-21 expression

miR-21 expression is monitored by real-time PCR as follows. Total RNA (including miRNAs) is prepared using the mirVana miRNA Isolation Kit (Ambion, Austin, TX, USA) according to the manufacturer's protocol. RNA quality and concentration are examined by the RNA 6000 LabChip Kit on the 2100 bioanalyzer (Agilent Technologies, Waldbronn, Germany).

To analyse levels of primary miR-21 transcripts, real-time PCR is performed using a LightCycler (Roche, Mannheim, Germany). Reverse transcriptase reacion is done with the SuperScript III First-Strand Synthesis System for RT-PCR (Invitrogen, Karlsruhe, Germany). An optimal PCR reaction is established by using the FastStart DNA Master SYBR Green I Kit (Roche, Mannheim, Germany). The identity of the PCR products is confirmed by sequencing.

Mature miR-21 is detected by real-time PCR with the TaqMan® MicroRNA Assay (Applied Biosystems (Foster Cits, CA, USA) using the Applied Biosystems 7900HT Fast Real-Time PCR System, according to the manufacturer's protocols.

### Example 5

### Strategy and methods suitable to determine efficacy of compounds with respect to miR-21 action

To analyse the efficacy of compounds targeting miR-21 itself and thereby blocking its pathogenetic effects, cellular systems are used as suited for the particular disease model under investigation. Compounds are administered to the cells by delivery methods optimised for the chosen cell system, e.g. by electroporation, lipofection, or other standard transfection techniques. Selected cell physiological consequences caused by that inhibition are monitored to determine the therapeutic potential of the test compounds. The parameters studied depend on the particular disease, e.g. for studies on the oncogenic potential of miR-21, it is determined whether successful down-regulation of miR-21 induces apoptosis and/or arrests the cell cycle of target cells. Apoptosis is detected by flow cytometry using annexin V and 7-amino-actinomycin D (7-AAD) staining as described (see reference 11). Likewise, cell cycle measurements are carried out by flow cytometry employing propidium iodide (see same reference).

### Further References:

1. Bromberg, J. F., M. H. Wrzeszczynska, G. Devgan, Y. Zhao, R. G. Pestell, C. Albanese, and J. E. Darnell, Jr. 1999. Stat3 as an oncogene. Cell 98:295*.*
2. Hodge, D. R., E. M. Hurt, and W. L. Farrar. 2005. The role of IL-6 and STAT3 in inflammation and cancer. Eur. J. Cancer*.*
3. Bromberg, J. F., C. M. Horvath, D. Besser, W. W. Lathem, and J. E. Darnell, Jr. 1998. Stat3 activation is required for cellular transformation by v-src. Mol. Cell. Biol. 18: 2553*.*
4. Gao, L., L. Zhang, J. Hu, F. Li, Y. Shao, D. Zhao, D. V. Kalvakolanu, D. J. Kopecko, X. Zhao, and D. Q. Xu. 2005. Down-regulation of signal transducer and activator of transcription 3 expression using vector-based small interfering RNAs suppresses growth of human prostate tumor in vivo. Clin. Cancer Res. 11:6333*.*
5. Kortylewski, M., R. Jove, and H. Yu. 2005. Targeting STAT3 affects melanoma on multiple fronts. Cancer Metastasis Rev. 24:315*.*
6. DeArmond, D., M. G. Brattain, J. M. Jessup, J. Kreisberg, S. Malik, S. Zhao, and J. W. Freeman. 2003. Autocrine-mediated ErbB-2 kinase activation of STAT3 is required for growth factor independence of pancreatic cancer cell lines. Oncogene 22: 7781*.*
7. Ling, X., and R. B. Arlinghaus. 2005. Knockdown of STAT3 expression by RNA interference inhibits the induction of breast tumors in immunocompetent mice. Cancer Res. 65: 2532*.*
8. Konnikova, L., M. Kotecki, M. M. Kruger, and B. H. Cochran. 2003. Knockdown of STAT3 expression by RNAi induces apoptosis in astrocytoma cells. BMC Cancer 3: 23*.*
9. Haura, E. B., J. Turkson, and R. Jove. 2005. Mechanisms of disease: Insights into the emerging role of signal transducers and activators of transcription in cancer. Nat. Clin. Pract. Oncol. 2:315*.*
10. Holtick, U., M. Vockerodt, D. Pinkert, N. Schoof, B. Sturzenhofecker, N. Kussebi, K. Lauber, S. Wesselborg, D. Löffler, F. Horn, L. Trümper, and D. Kube. 2005. STAT3 is essential for Hodgkin lymphoma cell proliferation and is a target of tyrphostin AG17 which confers sensitization for apoptosis. Leukemia 19:936*.*
11. Brocke-Heidrich, K., A. K. Kretzschmar, G. Pfeifer, C. Henze, D. Löffler, D. Koczan, H. J. Thiesen, R. Burger, M. Gramatzki, and F. Horn. 2004. Interleukin-6-dependent gene expression profiles in multiple myeloma INA-6 cells reveal a Bcl-2 family-independent survival pathway closely associated with Stat3 activation. Blood 103: 242*.*
12. Torres-Roca, J. F., M. DeSilvio, L. B. Mora, L. Y. Khor, E. Hammond, N. Ahmad, R. Jove, J. Forman, R. J. Lee, H. Sandler, and A. Pollack. 2007. Activated STAT3 as a correlate of distant metastasis in prostate cancer: a secondary analysis of Radiation Therapy Oncology Group 86-10. Urology 69:505*.*
13. Li, W. C., S. L. Ye, R. X. Sun, Y. K. Liu, Z. Y. Tang, Y. Kim, J. G. Karras, and H. Zhang. 2006. Inhibition of growth and metastasis of human hepatocellular carcinoma by antisense oligonucleotide targeting signal transducer and activator of transcription 3. Clin. Cancer Res. 12: 7140*.*
14. Xie, T. X., F. J. Huang, K. D. Aldape, S. H. Kang, M. Liu, J. E. Gershenwald, K. Xie, R. Sawaya, and S. Huang. 2006. Activation of stat3 in human melanoma promotes brain metastasis. Cancer Res. 66:3188*.*
15. Benekli, M., Z. Xia, K. A. Donohue, L. A. Ford, L. A. Pixley, M. R. Baer, H. Baumann, and M. Wetzler. 2002. Constitutive activity of signal transducer and activator of transcription 3 protein in acute myeloid leukemia blasts is associated with short disease-free survival. Blood 99:252*.*
16. Coppola, D. 2000. Molecular prognostic markers in pancreatic cancer. Cancer Control 7:421*.*
17. Kusaba, T., T. Nakayama, K. Yamazumi, Y. Yakata, A. Yoshizaki, K. Inoue, T. Nagayasu, and I. Sekine. 2006. Activation of STAT3 is a marker of poor prognosis in human colorectal cancer. Oncol Rep 15:1445*.*
18. Chang, K. C., M. H. Wu, D. Jones, F. F. Chen, and Y. L. Tseng. 2006. Activation of STAT3 in thymic epithelial tumours correlates with tumour type and clinical behaviour. J. Pathol. 210:224*.*
19. Pfitzner, E., S. Kliem, D. Baus, and C. M. Litterst. 2004. The role of STATs in inflammation and inflammatory diseases. Curr Pharm Des 10: 2839*.*
20. Ishihara, K., and T. Hirano. 2002. IL-6 in autoimmune disease and chronic inflammatory proliferative disease. Cytokine Growth Factor Rev. 13:357*.*
21. Krause, A., N. Scaletta, J. D. Ji, and L. B. Ivashkiv. 2002. Rheumatoid arthritis synoviocyte survival is dependent on Stat3. J. Immunol. 169:6610*.*
22. Barton, B. E. 2006. STAT3: a potential therapeutic target in dendritic cells for the induction of transplant tolerance. Expert Opin Ther Targets 10:459*.*
23. Secko, D. 2005. Cancer therapy finds a solid target. CMAJ 173:246.
24. Klampfer, L. 2006. Signal transducers and activators of transcription (STATs): Novel targets of chemopreventive and chemotherapeutic drugs. Curr. Cancer Drug Targets 6:107*.*
25. Bartel, D. P. 2004. MicroRNAs: genomics, biogenesis, mechanism, and function. Cell 116:281*.*
26. Hwang, H. W., and J. T. Mendell. 2006. MicroRNAs in cell proliferation, cell death, and tumorigenesis. Br. J. Cancer 94:776*.*
27. Xu, P., M. Guo, and B. A. Hay. 2004. MicroRNAs and the regulation of cell death. Trends Genet. 20:617*.*
28. Chen, C. Z., L. Li, H. F. Lodish, and D. P. Bartel. 2004. MicroRNAs modulate hematopoietic lineage differentiation. Science 303:83*.*
29. Lagos-Quintana, M., R. Rauhut, A. Yalcin, J. Meyer, W. Lendeckel, and T. Tuschl. 2002. Identification of tissue-specific microRNAs from mouse. Curr. Biol. 12:735*.*
30. Esquela-Kerscher, A., and F. J. Slack. 2006. Oncomirs - microRNAs with a role in cancer. Nat. Rev. Cancer 6:259*.*
31. He, L., J. M. Thomson, M. T. Hemann, E. Hernando-Monge, D. Mu, S. Goodson, S. Powers, C. Cordon-Cardo, S. W. Lowe, G. J. Hannon, and S. M. Hammond. 2005. A microRNA polycistron as a potential human oncogene. Nature 435:828*.*
32. Volinia, S., G. A. Calin, C. G. Liu, S. Ambs, A. Cimmino, F. Petrocca, R. Visone, M. Iorio, C. Roldo, M. Ferracin, R. L. Prueitt, N. Yanaihara, G. Lanza, A. Scarpa, A. Vecchione, M. Negrini, C. C. Harris, and C. M. Croce. 2006. A microRNA expression signature of human solid tumors defines cancer gene targets. Proc. Natl. Acad. Sci. USA 103:2257*.*
33. Iorio, M. V., M. Ferracin, C. G. Liu, A. Veronese, R. Spizzo, S. Sabbioni, E. Magri, M. Pedriali, M. Fabbri, M. Campiglio, S. Menard, J. P. Palazzo, A. Rosenberg, P. Musiani, S. Volinia, I. Nenci, G. A. Calin, P. Querzoli, M. Negrini, and C. M. Croce. 2005. MicroRNA gene expression deregulation in human breast cancer. Cancer Res. 65:7065*.*
34. Chan, J. A., A. M. Krichevsky, and K. S. Kosik. 2005. MicroRNA-21 is an antiapoptotic factor in human glioblastoma cells. Cancer Res. 65:6029*.*
35. Cheng, A. M., M. W. Byrom, J. Shelton, and L. P. Ford. 2005. Antisense inhibition of human miRNAs and indications for an involvement of miRNA in cell growth and apoptosis. Nucleic Acids Res. 33:1290*.*
36. Meng, F., R. Henson, M. Lang, H. Wehbe, S. Maheshwari, J. T. Mendell, J. Jiang, T. D. Schmittgen, and T. Patel. 2006. Involvement of human micro-RNA in growth and response to chemotherapy in human cholangiocarcinoma cell lines. Gastroenterology 130:2113*.*
37. Castanotto, D., S. Tommasi, M. Li, H. Li, S. Yanow, G. P. Pfeifer, and J. J. Rossi. 2005. Short hairpin RNA-directed cytosine (CpG) methylation of the RASSF1A gene promoter in HeLa cells. Mol. Ther. 12:179*.*
38. Ting, A. H., K. E. Schuebel, J. G. Herman, and S. B. Baylin. 2005. Short double-stranded RNA induces transcriptional gene silencing in human cancer cells in the absence of DNA methylation. Nat. Genet. 37:906*.*
39. Kawasaki, H., and K. Taira. 2004. Induction of DNA methylation and gene silencing by short interfering RNAs in human cells. Nature 431:211*.*
40. Morris, K. V., S. W. Chan, S. E. Jacobsen, and D. J. Looney. 2004. Small interfering RNA-induced transcriptional gene silencing in human cells. Science 305:1289*.*
41. Weinberg, M. S., L. M. Villeneuve, A. Ehsani, M. Amarzguioui, L. Aagaard, Z. X. Chen, A. D. Riggs, J. J. Rossi, and K. V. Morris. 2006. The antisense strand of small interfering RNAs directs histone methylation and transcriptional gene silencing in human cells. RNA 12:256*.*
42. Altschul, S. F., W. Gish, W. Miller, E. W. Myers, and D. J. Lipman. 1990. Basic local alignment search tool. J. Mol. Biol. 215:403*.*
43. Altschul, S. F., and W. Gish. 1996. Local alignment statistics. Methods Enzymol. 266:460*.*
44. Pearson, W. R., and D. J. Lipman. 1988. Improved tools for biological sequence comparison. Proc. Natl. Acad. Sci. USA 85:2444*.*
45. Smith, T. F., and M. S. Waterman. 1981. Identification of common molecular subsequences. J. Mol. Biol. 147:195*.*
46. Thompson, J. D., D. G. Higgins, and T. J. Gibson. 1994. CLUSTAL W: improving the sensitivity of progressive multiple sequence alignment through sequence weighting, position-specific gap penalties and weight matrix choice. Nucleic Acids Res. 22:4673*.*
47. Catlett-Falcone, R., T. H. Landowski, M. M. Oshiro, J. Turkson, A. Levitzki, R. Savino, G. Ciliberto, L. Moscinski, J. L. Fernandez-Luna, G. Nunez, W. S. Dalton, and R. Jove. 1999. Constitutive activation of Stat3 signaling confers resistance to apoptosis in human U266 myeloma cells. Immunity 10:105*.*
48. Kawano, M. M., H. Ishikawa, N. Tsuyama, S. Abroun, S. Liu, F. J. Li, K. Otsuyama, and X. Zheng. 2002. Growth mechanism of human myeloma cells by interleukin-6. Int. J. Hematol. 76:329*.*
49. Braasch, D. A., and D. R. Corey. 2001. Locked nucleic acid (LNA): fine-tuning the recognition of DNA and RNA. Chem. Biol. 8:1*.*
50. Nielsen, P. E., M. Egholm, R. H. Berg, and O. Buchardt. 1991. Sequence-selective recognition of DNA by strand displacement with a thymine-substituted polyamide. Science 254:1497*.*
51. Egholm, M., O. Buchardt, L. Christensen, C. Behrens, S. M. Freier, D. A. Driver, R. H. Berg, S. K. Kim, B. Norden, and P. E. Nielsen. 1993. PNA hybridizes to complementary oligonucleotides obeying the Watson-Crick hydrogen-bonding rules. Nature 365:566*.*
52. Demidov, V. V., V. N. Potaman, M. D. Frank-Kamenetskii, M. Egholm, O. Buchard, S. H. Sonnichsen, and P. E. Nielsen. 1994. Stability of peptide nucleic acids in human serum and cellular extracts. Biochem Pharmacol 48:1310*.*
53. Wittung, P., P. E. Nielsen, O. Buchardt, M. Egholm, and B. Norden. 1994. DNA-like double helix formed by peptide nucleic acid. Nature 368:561*.*
54. Ray, A., and B. Norden. 2000. Peptide nucleic acid (PNA): its medical and biotechnical applications and promise for the future. FASEB J. 14:1041*.*
55. Melani, C., L. Rivoltini, G. Parmiani, B. Calabretta, and M. P. Colombo. 1991. Inhibition of proliferation by c-myb antisense oligodeoxynucleotides in colon adenocarcinoma cell lines that express c-myb. Cancer Res. 51:2897*.*
56. Zamore, P. D. 2001. RNA interference: listening to the sound of silence. Nat. Struct. Biol. 8:746*.*
57. Tuschl, T. 2001. RNA interference and small interfering RNAs. Chembiochem 2:239*.*
58. Steinecke, P., and P. H. Schreier. 1995. Ribozymes. Methods Cell Biol. 50:449*.*
59. Kel, A. E., E. Gossling, 1. Reuter, E. Cheremushkin, O. V. Kel-Margoulis, and E. Wingender. 2003. MATCH: A tool for searching transcription factor binding sites in DNA sequences. Nucleic Acids Res. 31:3576*.*
60. Siepel, A., G. Bejerano, J. S. Pedersen, A. S. Hinrichs, M. Hou, K. Rosenbloom, H. Clawson, J. Spieth, L. W. Hillier, S. Richards, G. M. Weinstock, R. K. Wilson, R. A. Gibbs, W. J. Kent, W. Miller, and D. Haussler. 2005. Evolutionarily conserved elements in vertebrate, insect, worm, and yeast genomes. Genome Res. 15:1034*.*
61. Wegenka, U. M., J. Buschmann, C. Lütticken, P. C. Heinrich, and F. Horn. 1993. Acute-phase response factor, a nuclear factor binding to acute-phase response elements, is rapidly activated by interleukin-6 at the posttranslational level. Mol. Cell. Biol. 13:276*.*
62. Yuan, J., U. M. Wegenka, C. Lütticken, J. Buschmann, T. Decker, C. Schindler, P. C. Heinrich, and F. Horn. 1994. The signalling pathways of interleukin-6 and gamma interferon converge by the activation of different transcription factors which bind to common responsive DNA elements. Mol. Cell. Biol. 14:1657*.*
63. Prohaska, S. J., C. Fried, C. Flamm, G. P. Wagner, and P. F. Stadler. 2004. Surveying phylogenetic footprints in large gene clusters: applications to Hox cluster duplications. Mol. Phylogenet. Evol. 31:581*.*
64. Chen, C., D. A. Ridzon, A. J. Broomer, Z. Zhou, D. H. Lee, J. T. Nguyen, M. Barbisin, N. L. Xu, V. R. Mahuvakar, M. R. Andersen, K. Q. Lao, K. J. Livak, and K. J. Guegler. 2005. Real-time quantification of microRNAs by stem-loop RT-PCR. Nucleic Acids Res. 33:e179*.*
65. Griffiths-Jones, S. 2004. The microRNA Registry. Nucleic Acids Res. 32:D109*.*
66. Griffiths-Jones, S., S. Moxon, M. Marshall, A. Khanna, S. R. Eddy, and A. Bateman. 2005. Rfam: annotating non-coding RNAs in complete genomes. Nucleic Acids Res. 33:D121*.*
67. Santini, S., J. L. Boore, and A. Meyer. 2003. Evolutionary conservation of regulatory elements in vertebrate Hox gene clusters. Genome Res. 13: 1111*.*
68. Cai, X., C. H. Hagedorn, and B. R. Cullen. 2004. Human microRNAs are processed from capped, polyadenylated transcripts that can also function as mRNAs. RNA 10:1957*.*
69. Rahaman, S. O., P. C. Harbor, O. Chernova, G. H. Barnett, M. A. Vogelbaum, and S. J. Haque. 2002. Inhibition of constitutively active Stat3 suppresses proliferation and induces apoptosis in glioblastoma multiforme cells. Oncogene 21:8404*.*
70. Isomoto, H., S. Kobayashi, N. W. Werneburg, S. F. Bronk, M. E. Guicciardi, D. A. Frank, and G. J. Gores. 2005. Interleukin 6 upregulates myeloid cell leukemia-1 expression through a STAT3 pathway in cholangiocarcinoma cells. Hepatology 42:1329*.*
71. Aoki, Y., G. M. Feldman, and G. Tosato. 2003. Inhibition of STAT3 signaling induces apoptosis and decreases survivin expression in primary effusion lymphoma. Blood 101:1535*.*
72. Jourdan, M., J. L. Veyrune, J. D. Vos, N. Redal, G. Couderc, and B. Klein. 2003. A major role for Mcl-1 antiapoptotic protein in the IL-6-induced survival of human myeloma cells. Oncogene 22:2950*.*
73. Zhang, B., 1. Gojo, and R. G. Fenton. 2002. Myeloid cell factor-1 is a critical survival factor for multiple myeloma. Blood 99:1885*.*
74. Derenne, S., B. Monia, N. M. Dean, J. K. Taylor, M. J. Rapp, J. L. Harousseau, R. Bataille, and M. Amiot. 2002. Antisense strategy shows that Mcl-1 rather than Bcl-2 or Bcl-x(L) is an essential survival protein of human myeloma cells. Blood 100:194*.*
75. Tumang, J. R., C. Y. Hsia, W. Tian, J. F. Bromberg, and H. C. Liou. 2002. IL-6 rescues the hyporesponsiveness of c-Rel deficient B cells independent of Bcl-xL, Mcl-1, and Bcl-2. Cell. Immunol. 217:47*.*
76. Miyagi, C., S. Yamashita, Y. Ohba, H. Yoshizaki, M. Matsuda, and T. Hirano. 2004. STAT3 noncell-autonomously controls planar cell polarity during zebrafish convergence and extension. J. Cell. Biol. 166:975*.*
77. Yamashita, S., C. Miyagi, A. Carmany-Rampey, T. Shimizu, R. Fujii, A. F. Schier, and T. Hirano. 2002. Stat3 Controls Cell Movements during Zebrafish Gastrulation. Dev. Cel12:363*.*

## Claims

1. A compound, wherein said compound is
(a) a polynucleotide comprising or consisting of a base sequence of contiguous bases from the sequence of any one of SEQ ID NOs: 1 to 5, said base sequence being at least 15 bases in length;
(b) a polynucleotide having at least 80% sequence identity to the polynucleotide of (a) over the entire length of said base sequence;
(c) an analog or derivative of (a) or (b); or
(d) a polynucleotide or analog or derivative thereof which is complementary to the full length of any one of (a) to (c);
wherein said polynucleotide having at least 80% sequence identity, said analog, or said derivative is capable of interacting with a nucleic acid comprising a double-stranded part, said part comprising the sequence of any one of SEQ ID NOs: 1 to 5.

2. The compound of claim 1, wherein said base sequence is not more than 50 bases in length, preferably between 20 and 25 bases, most preferred 22 bases.

3. A pharmaceutical composition comprising
(a) one or more compounds of claim 1 or 2; and/or
(b) an inhibitor of the interaction between Stat3 and a nucleic acid as defined in claim 1.

4. Use of one or more compounds of claim 1 or 2 or of the inhibitor defined in claim 3 for the manufacture of a pharmaceutical composition for the treatment of cancer, inflammatory diseases, autoimmune diseases, or a disease with an involvement of Stat3 activation for aetiology and/or progression.

5. A method of identifying a compound suitable for the treatment of cancer, inflammatory diseases, autoimmune diseases, or a disease with an involvement of Stat3 activation for aetiology and/or progression; or suitable as a lead compound for the development of such compound suitable for treatment; the method comprising the steps of:
(a) bringing into contact Stat3; a test compound; and a nucleic acid comprising a double-stranded part, said part comprising the sequence of any one of SEQ ID NOs 1 to 5;
(b) determining
(i) the amount of complex formed between Stat3 and said nucleic acid;
(ii) modifications of said nucleic acid and/or of histones, if any, bound thereto, said modifications being indicative of silencing; and/or
(iii) the amount of transcript of a gene being under the control of said nucleic acid;
in the presence of said test compound; and
(c) comparing
(i) the amount determined in step (b)(i) with the amount of said complex formed in absence of said test compound;
(ii) modifications determined in step (b)(ii) with the modifications in absence of said test compound; and/or
(iii) the amount determined in step (b)(iii) with the amount of transcript of said gene in absence of said test compound;
wherein a lower amount determined in step (b)(i); more or different modifications determined in step (b)(ii); and/or a lower amount of transcript determined in step (b)(iii) is/are indicative of a compound suitable for treatment or as a lead compound.

6. The method of claim 5, wherein said double-stranded part is double-stranded DNA and said modifications are methylation of said DNA or acetylation, methylation, or phosphorylation of histones bound to said DNA.

7. The method of claim 5 or 6, further comprising the steps of:
(d) bringing into contact Stat3; said test compound; and a nucleic acid comprising a double-stranded part, said part comprising the sequence of the Stat3-binding region of a gene selected from the group consisting of haptoglobin, ICAM1, SERPINA3, SOCS2 and BCL 3;
(e) determining
(i) the amount of complex formed between Stat3 and said nucleic acid defined in (d);
(ii) modifications of said nucleic acid defined in (d) and/or of histones, if any, bound thereto, said modifications being indicative of silencing; and/or
(iii) the amount of transcript of a gene being under the control of said nucleic acid defined in (d);
in the presence of said test compound; and
(f) comparing
(i) the amount determined in step (e)(i) with the amount of said complex formed in absence of said test compound;
(ii) modifications determined in step (e)(ii) with the modifications in absence of said test compound; and/or
(iii) the amount determined in step (e)(iii) with the amount of transcript of said gene in absence of said test compound;
wherein equal amounts of the complex as defined in (e)(i), identical modifications or equal numbers of modifications, said modifications being defined in (e)(ii), and/or equal amounts of transcript as defined in (e)(iii) are indicative of a test compound not interfering with the interaction of Stat3 with said nucleic acid defined in (d).

8. Use of a nucleic acid, analog or derivative thereof, wherein said nucleic acid, analog or derivative thereof consists of or comprises a base sequence, said base sequence
(a) being complementary to the full length of the sequence of SEQ ID NO: 6;
(b) being capable of hybridising under stringent conditions with the sequence of SEQ ID NO: 6; or
(c) having at least 80% sequence identity to the sequence of (a); for the manufacture of a pharmaceutical composition for the treatment of inflammatory diseases, autoimmune diseases, acute myeloid leukaemia, anaplastic large cell lymphoma, cholangiocarcinoma, colon carcinoma, head and neck cancer, hepatocellular carcinoma, hodgkin lymphoma, lung cancer, melanoma, multiple myeloma, pancreas carcinoma, prostate carcinoma and thymic epithelial tumours, or a disease with an involvement of Stat3 activation for aetiology and/or progression.

9. An in vitro or ex vivo method of diagnosing
(a) a disease or condition selected from the group consisting of inflammatory diseases, autoimmune diseases, multiple myeloma, prostate carcinoma, and diseases with an involvement of Stat3 activation for aetiology and/or progression; or
(b) subtypes of such disease exhibiting different prognosis or treatment responsiveness, the method comprising
determining in a sample obtained from a subject the amount of nucleic acid consisting of or comprising (i) the sequence of SEQ ID NO: 6; or (ii) a sequence having at least 80% sequence identity to the sequence of SEQ ID NO: 6 over the entire length of the sequence of SEQ ID NO: 6,
wherein a higher amount as compared to a sample obtained from a healthy subject is indicative
(a) of said disease or condition; or
(b) of worse prognosis or treatment responsiveness.

10. The use of claim 4 or 8 or the method of any one of claims 5 to 7 or 9, wherein said inflammatory disease or autoimmune disease is selected from the group consisting of asthma, autoimmune diabetes, colitis, crohn disease, glomerulonephritis, inflammatory bowel disease, multiple sclerosis, osteoporosis, pancreatitis, psoriasis, rheumatoid arthritis, systemic lupus erythematosus and systemic-onset juvenile chronic arthritis.

11. The use of claim 4 or 8 or the method of any one of claims 5 to 7 or 9, wherein said disease with an involvement of Stat3 activation for aetiology and/or progression is a cancer with an involvement of Stat3 activation for aetiology and/or progression.

12. The use or the method of claim 11, wherein said cancer with an involvement of Stat3 activation for aetiology and/or progression is selected from the group consisting of Stat3-dependent acute myeloid leukaemia, Stat3-dependent anaplastic large cell lymphoma, Stat3-dependent cholangiocarcinoma, Stat3-dependent colon carcinoma, Stat3-dependent glioblastoma, Stat3-dependent head and neck cancer, Stat3-dependent hepatocellular carcinoma, Stat3-dependent Hodgkin lymphoma, Stat3-dependent lung cancer, Stat3-dependent mammary carcinoma, Stat3-dependent melanoma, Stat3-dependent multiple myeloma, Stat3-dependent pancreas carcinoma, Stat3-dependent prostate carcinoma and Stat3-dependent thymic epithelial tumours.

13. The compound of claim 1 or 2, the pharmaceutical composition of claim 3, or the use of any one of claims 4, 8 or 10 to 12, wherein said analog or derivative is selected from the group consisting of peptide nucleic acids, phosphorothioate nucleic acids, phosphoramidate nucleic acids, 2'-O-methoxyethyl ribonucleic acids, morpholino nucleic acids, hexitol nucleic acids, locked nucleic acids, chimeras thereof and chimeras of oligonucleotides or polynucleotides and any one of peptide nucleic acids, phosphorothioate nucleic acids, phosphoramidate nucleic acids, 2'-O-methoxyethyl ribonucleic acids, morpholino nucleic acids, hexitol nucleic acids and locked nucleic acids.

14. The compound of claim 1 or 2, the pharmaceutical composition of claim 3, or use of any one of claims 4, 8 or 10 to 13, wherein said polynucleotide, nucleic acid, analog or derivative thereof is an antisense molecule, an siRNA, or a ribozyme, comprising said base sequence.

15. The pharmaceutical composition of claim 3, the use of any one of claims 4, 8 or 10 to 14, or the method of any one of claims 5 to 7, 9, 11 or 12, wherein Stat3 is human Stat3


SEQUENCE LISTING

<110> Fraunhofer-Gesellschaft zur Förderung der angewandten Forschung e.V.

<120> MEANS FOR TREATING INFLAMMATORY DISEASES, AUTOIMMUNE DISEASES AND
CANCER

<130> M2716 EP

<160> 17

<170> PatentIn version 3.3

<210> 1
<211> 40
<212> DNA
<213> artificial sequence

<220>
<221> source
<223> /note="Description of artificial sequence: enhancer region"

<220>
<221> variation
<222> 10
<223> /replace="t"
/replace="c"
/replace="g"

<220>
<221> variation
<222> 11
<223> /replace="t"
/replace="c"
/replace="g"

<220>
<221> variation
<222> 12
<223> /replace="t"
/replace="c"
/replace="g"

<220>
<221> variation
<222> 13
<223> /replace="t"
/replace="c"
/replace="g"

<220>
<221> variation
<222> 14
<223> /replace="t"
/replace="c"
/replace="g"

<220>
<221> variation
<222> 15
<223> /replace="t"
/replace="c"
/replace="g"

<220>
<221>  variation
<222>  16
<223>  /replace="t"
       /replace="c"
       /replace="g"

<220>
<221>  variation
<222>  17
<223>  /replace="t"
       /replace="c"
       /replace="g"

<220>
<221>  variation
<222>  18
<223>  /replace="t"
       /replace="c"
       /replace="g"

<220>
<221>  variation
<222>  19
<223>  /replace="t"
       /replace="c"
       /replace="g"

<220>
<221>  variation
<222>  20
<223>  /replace="t"
       /replace="c"
       /replace="g"

<220>
<221>  variation
<222>  21
<223>  /replace="t"
       /replace="c"
       /replace="g"

<220>
<221>  variation
<222>  22
<223>  /replace="t"
       /replace="c"
       /replace="g"

<220>
<221>  variation
<222>  23
<223>  /replace="t"
       /replace="c"
       /replace="g"

<220>
<221>  variation
<222>  24
<223>  /replace="t"
       /replace="c"
       /replace="g"

<220>

<221>  variation
<222>  25
<223>  /replace="t"
       /replace="c"
       /replace="g"

<220>
<221>  variation
<222>  26
<223>  /replace="t"
       /replace="c"
       /replace="g"

<220>
<221>  variation
<222>  27
<223>  /replace="t"
       /replace="c"
       /replace="g"

<220>
<221>  variation
<222>  28
<223>  /replace="t"
       /replace="c"
       /replace="g"

<220>
<221>  variation
<222>  29
<223>  /replace="t"
       /replace="c"
       /replace="g"

<220>
<221>  variation
<222>  30
<223>  /replace="t"
       /replace="c"
       /replace="g"

<220>
<221>  variation
<222>  31
<223>  /replace="t"
       /replace="c"
       /replace="g"

<400>  1
ttacaggaaa aaaaaaaaaa aaaaaaaaaa attctgagaa                              40


<210>  2
<211>  40
<212>  DNA
<213>  homo sapiens

<400>  2
ttacaggaac tagtggtgat aaatgtggga cttctgagaa                            40


<210>  3
<211>  49
<212>  DNA

<213>  homo sapiens

<400>  3
taattggttc aaaccagttc ttacaggaac tagtggtgat aaatgtggg                49


<210>  4
<211>  49
<212>  DNA
<213>  homo sapiens

<400>  4
agtggtgata aatgtgggac ttctgagaag tcattcattt tattctttg                49


<210>  5
<211>  415
<212>  DNA
<213>  homo sapiens

<400>  5
ttatcaaaga tcactatccc aatcatctca gaacaagctg ttactaatgt actggagttt      60

ctgtgcaaac tgtctaccat aaaccatgaa aggattcaaa gttcatagtt ccttctttgt     120

tcctttgtta atcactgact tctgactagt gggaggtgcc tcccaagttt gctaatgcat     180

tctttttgga taaggatgac gcacagattg tcctaataag gacttagatt gagaaagacc     240

gccccctctg agaagagggg acaagtcaga gagagggcgg gcagtttctt ttttaactag     300

ggatgacaca agcataagtc atttccttat taattggttc aaaccagttc ttacaggaac     360

tagtggtgat aaatgtggga cttctgagaa gtcattcatt ttattctttg tgcca          415


<210>  6
<211>  22
<212>  RNA
<213>  homo sapiens

<400>  6
uagcuuauca gacugauguu ga                                              22


<210>  7
<211>  770
<212>  PRT
<213>  homo sapiens

<400>  7

<210>  8
<211>  49
<212>  DNA
<213>  homo sapiens

<400>  8
gggtattgtt tgtggttttg ttactggaaa agatagtgac cttaccagg                49

<210> 9
<211> 200
<212> DNA
<213> homo sapiens

<400> 9
catagagttg ccaggtttct tgggatttgt aatagaacat cacaagaaaa tcaagtgtga 60

agcaagagct caactcttaa caggggtatt gtttgtggtt ttgttactgg aaaagatagt 120

gaccttacca gggccaaagt ttgtagacac aggaattacg aaatggagaa gggggagaag 180

tgagctagtg gcagcataaa 200


<210> 10
<211> 49
<212> DNA
<213> homo sapiens

<400> 10
cggaggggaa ggcgcgaggt ttccgggaaa gcagcaccgc cccttggcc 49


<210> 11
<211> 200
<212> DNA
<213> homo sapiens

<400> 11
gggtcatcgc cctgccaccg ccgcccgatt gctttagctt ggaaattccg gagctgaagc 60

ggccagcgag ggaggatgac cctctcggcc cgggcaccct gtcagtccgg aaataactgc 120

agcatttgtt ccggagggga aggcgcgagg tttccgggaa agcagcaccg ccccttggcc 180

cccaggtggc tagcgctata 200


<210> 12
<211> 39
<212> DNA
<213> homo sapiens

<400> 12
ttaccagaaa ttatcatctg gtcatttcca gtccgagaa 39


<210> 13
<211> 200
<212> DNA
<213> homo sapiens

<400> 13
tttttctgtg tgcttttttg acctttggag gaagagatta gagctagtcc cataaccagg 60

ttatttgagt aggtctaaca agcccgtatt accagaaatt atcatctggt catttccagt 120

ccgagaacag aacacttggt tgtcctggca tttcccaagc agggggagga gttctctgca 180

ggaataaata agcctcagca 200

<210> 14
<211> 271
<212> DNA
<213> homo sapiens

<400> 14
ttcttgtaat gtttagtcac tactcgcagc agagaaaggc tgagcgcgga gggtgaggga 60

ggggccgcgg gcgccgaggg cgggccgcga gcaggggcag ggaccgggag ggacgcgcgc 120

ggagacaaag cgcgacgaga ggcggccgag gcggccggtg ggcgcggcgc cagccccggc 180

ggcgcgttcc tggcagcggc ccctccccgc cgcgcgctcc gcccccaact tctcattcac 240

actttccccc cacccccctc ccttctaaga a 271


<210> 15
<211> 350
<212> DNA
<213> homo sapiens

<400> 15
ccccgattcc tggaactgcg cggccggcct tcttgtaatg tttagtcact actcgcagca 60

gagaaaggct gagcgcggag ggtgagggag gggccgcggg cgccgagggc gggccgcgag 120

caggggcagg gaccgggagg gacgcgcgcg gagacaaagc gcgacgagag gcggccgagg 180

cggccggtgg gcgcggcgcc agccccggcg gcgcgttcct ggcagcggcc cctccccgcc 240

gcgcgctccg cccccaactt ctcattcaca ctttcccccc acccccctcc cttctaagaa 300

ggctgatttc tggcagaggc gggggcgccg cttcgggaga gcgggcagtt 350


<210> 16
<211> 114
<212> DNA
<213> homo sapiens

<400> 16
ttactggaag tccgagggct gggtcgcccc tcccgtcccc acttcctcca gccccgcccc 60

accccctgcg gcccgcgcgt ttatgggaac tcaggcctgg acggtttctc tgaa 114


<210> 17
<211> 200
<212> DNA
<213> homo sapiens

<400> 17
cttccctccc ccgggagcag gaagcagagc cccgcccagc agacctgtta ctggaagtcc 60

gagggctggg tcgcccctcc cgtccccact tcctccagcc ccgccccacc ccctgcggcc 120

cgcgcgttta tgggaactca ggcctggacg gtttctctga aatcattcgc tgcgtctgcc 180

ctcacttaac cctggccgcc 200